(19) Europäisches Patentamt
European Patent Office
Office européen des brevets

(11) EP 4 554 563 B1

(12) # EUROPEAN PATENT SPECIFICATION

(45) Date of publication and mention of the grant of the patent:
**25.02.2026 Bulletin 2026/09**

(21) Application number: **23741394.3**

(22) Date of filing: **12.07.2023**

(51) International Patent Classification (IPC):
***A61K 9/107*** *(2006.01)* ***A61P 1/00*** *(2006.01)*
***A61K 47/50*** *(2017.01)* ***A61K 9/00*** *(2006.01)*
***A61K 38/43*** *(2006.01)* ***A61K 38/47*** *(2006.01)*
***A61K 47/59*** *(2017.01)* ***A61K 47/62*** *(2017.01)*
***A61K 47/69*** *(2017.01)* ***A61K 38/46*** *(2006.01)*
***A61K 38/39*** *(2006.01)* ***A61K 38/19*** *(2006.01)*
***A61K 39/395*** *(2006.01)*

(52) Cooperative Patent Classification (CPC):
**A61K 9/1075; A61K 9/0019; A61K 38/193; A61K 38/43; A61K 38/465; A61K 38/47; A61K 39/39591; A61K 47/593; A61K 47/62; A61K 47/6909; C12Y 301/06013; C12Y 302/01046**

(86) International application number:
**PCT/EP2023/069351**

(87) International publication number:
**WO 2024/013245 (18.01.2024 Gazette 2024/03)**

(54) **REVERSED MICELLES FOR DELIVERY OF HYDROPHILIC DRUGS**

UMGEKEHRTE MIZELLEN ZUR ABGABE HYDROPHILER ARZNEIMITTEL

MICELLES INVERSÉES POUR L'ADMINISTRATION DE MÉDICAMENTS HYDROPHILES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **14.07.2022 IT 202200014791**

(43) Date of publication of application:
**21.05.2025 Bulletin 2025/21**

(73) Proprietor: **Consiglio Nazionale Delle Ricerche - CNR**
**00185 Roma (IT)**

(72) Inventors:
- **CECCHINI, Marco**
  **000185 Roma (RM) (IT)**
- **GAGLIARDI, Mariacristina**
  **00185 Roma (RM) (IT)**

(74) Representative: **Currado, Luisa et al**
**De Simone & Partners S.r.l.**
**Via Giulio Caccini, 1**
**00198 Roma (IT)**

(56) References cited:
**WO-A1-2021/046078 CN-B- 105 524 272**

- **XU YANMIN ET AL, vol. 9, no. 12, 10 November 2009 (2009-11-10), DE, pages 1254 - 1261, XP055814481, ISSN: 1616-5187, Retrieved from the Internet <URL:https://api.wiley.com/onlinelibrary/tdm/v1/articles/10.1002%2Fmabi.200900233> DOI: 10.1002/mabi.200900233**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 554 563 B1

## Description

### FIELD OF THE INVENTION

The present invention refers to the field of nanosystems for drug delivery, in particular it refers to micelles for delivery of hydrophilic macromolecules.

### BACKGROUND OF THE INVENTION

The systemic administration of active molecules has some limitations which, generally, result in a poor efficacy of the pharmacological treatment.

In central nervous system treatments, the penetration and deposition of drugs are also hindered by the blood brain barrier (BBB), which prevents the entry of a large number of molecules. Large molecules, such as proteins and some peptides and enzymes, in the absence of a specific transporter are completely blocked by the BBB.

However, some pathologies, as in the case of certain cancers (e.g. glioblastoma multiforme), neurodegenerative diseases (e.g. Parkinson's disease), or some genetic diseases (e.g. leukodystrophies), require the distribution of the active ingredient in the brain.

To date, effective and minimally invasive treatments for such pathologies are not widely available.

Some strategies are currently under investigation for the translocation of drugs into the brain through the use of peptides or virus fragments conjugated to the drug of interest or to a nanovector. Most of the nanocarriers developed for this purpose are composed of mesoporous silica, lipids or solid polymeric nanoparticles. The use of a nanovector represents a highly effective strategy for small to medium-sized molecules, easily trapped in the vector.

However, macromolecules have a large steric footprint, so their highly efficient entrapment in solid vectors can be difficult to achieve. Macromolecules are generally efficiently trapped in structures that can be assembled around them. The self-assembly of the nanocarrier around the active principle can be obtained by complexation, when the molecules involved are both electrically charged, or by hydrophilic/hydrophobic interactions.

Micelles are structures obtained by self-assembly. Micelles are generally obtained with an oil-in-water emulsion, using organic molecules with a localized charge, such as an ion, which is hydrophilic, and a hydrocarbon tail, which is hydrophobic. Micelles are generally used to solubilize hydrophobic molecules in an aqueous environment, forming a structure with a hydrophobic core and a hydrophilic shell. When micelles are made using a water-in-oil emulsion, their structure is reversed, with a hydrophilic core and a hydrophobic shell. This type of structure can be used to trap hydrophilic molecules.

In particular, polymer micelles composed of degradable and biocompatible materials are of great interest due to their biocompatibility. Polymers capable of forming micelles are referred to as amphiphilic, i.e. composed of two or more blocks with different degrees of hydrophilicity. A water-in-oil emulsion for obtaining reversed polymer micelles has been shown to be effective to obtain reverse micelles able encapsulate and release protein drugs (Koyamatzu et al., 2014); however, such structures are suitable only for oral drug delivery and not for systemic administration since they show poor stability when resuspended for systemic administration or in the bloodstream. Furthermore, the outer hydrophobic shell is very prone to forming the so-called protein crown, induced by the precipitation of blood proteins on the surface of the micelles.

Further reversed micelles for the delivery of drugs or other substances are known, for example from WO2012105485A1, CN104004199A, CN103272237A, CN110522720A, US2010/0196482 A1. In particular, WO2012105485A1, CN104004199A, CN103272237A disclose the use of polymeric reverse micelles. WO 2021/046078 A1 discloses reverse micelles that comprise copolymers of dextran with PLA or PLGA encapsulating hydrophilic compounds, for example RNA.

However, the reverse polymeric micelles known in the art are not sufficiently stable to be loaded with a macromolecule, such as a protein drug, and be used in an aqueous environment, such as a body fluid.

Some chemical strategies for the stabilization of micelles, mainly based on crosslinking, have been developed (see Xu et al., 2009; Heffernan and Murthy, 2009; Huang et al., 2017) but they have been applied only to non-polymeric or non-reversed micelles.

Therefore, there is still the need of an efficient mean to deliver systemically administered hydrophilic drugs, in particular large-sized drugs, to the target region of interest, especially for drugs that should cross the blood brain barrier.

It has now been found a nanocarrier able to transport hydrophilic active ingredients administered systemically to a specific body area. Said nanocarrier is a polymeric reverse micelle composed of an amphiphilic polymeric material which is chemically stabilized by means of a controlled cross-linking involving specific sites of the polymer. The obtained stabilized structure is advantageously able to encapsulate hydrophilic molecules and to transport them to the desired pathological site.

## SUMMARY OF THE INVENTION

It is an object of the invention a polymeric reverse micelle for the delivery of an active pharmaceutical ingredient, wherein the interior of the reverse micelle is hydrophilic and is suitable to contain a water-soluble active pharmaceutical ingredient and the exterior of the reverse micelle is hydrophobic, wherein said reverse micelle comprises at least two polymers, each one having the following formula (I):

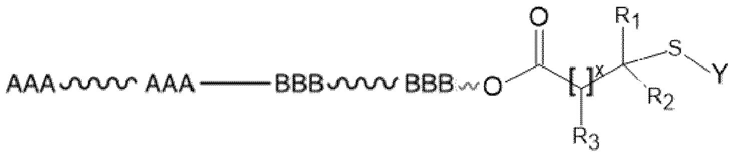

(I)

wherein

AAA ∿∿ AAA is an hydrophilic polymer covalently linked to BBB ∿∿ BBB which is an hydrophobic polymer,

R1, R2, R3, the same or different from each other, are selected from hydrogen, $C_{1-20}$ alkyl and $C_{1-20}$alkyl-O-$C_{1-20}$alkyl, preferably they are independently selected from methyl, ethyl, n-propyl or isopropyl;

x is 1 or 2;

Y is an alkyl chain substituted with at least two molecules of general formula (A):

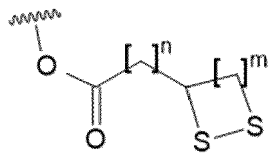

(A)

wherein n is comprised between 0 and 6;

m is comprised between 0 and 5;

said Y alkyl chain being optionally terminally substituted with SH;

and wherein said at least two polymers of formula (I) are linked through disulfide bonds between the thiol groups formed upon reduction of the -S-S- linkage in molecule (A),

wherein the percentage of thiol groups involved in inter-polymer disulfide bonds with respect to the total of the thiol groups present in molecules (A) is comprised between 70 and 100%, preferably between 90 and 100%.

In an embodiment, the micelle also comprises at least one pharmaceutical active ingredient.

In a preferred embodiment, the micelle of the invention further comprises one or more polymers of formula (III):

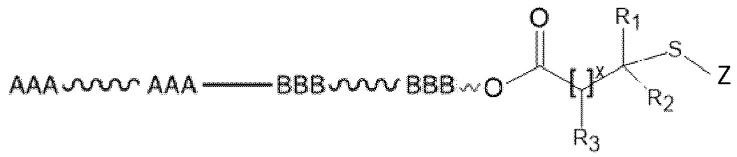

(III)

wherein AAA, BBB, R1, R2, R3 and x are as defined above, and wherein Z is an alkyl chain substituted with at least two hydroxyl groups and terminally conjugated to at least one targeting molecule.

Optionally, the polymer of formula (III) further comprises a linker L between the alkyl chain Z and the conjugated targeting molecule. Said linker L can be a molecule bearing at least one alkylidene and/or vinyl group. For example it can be trimethylolpropanetrimethacrylate (TRIM).

It is a further object of the invention a process to obtain a reverse micelle as above defined comprising at least the following steps:

a) reacting at least two polymers, each polymer comprising a hydrophilic polymer covalently linked to an hydrophobic polymer, said hydrophobic polymer provided with a terminal hydroxyl group, with an alkenyl halide thus obtaining polymers with an - ene terminal functional group;
b) reacting the obtained polymers with a thiopolyol to obtain polymers with at least two hydroxyl lateral groups;
c) conjugating each of said hydroxyl lateral groups of the polymers with a cyclic disulfide of formula (A')

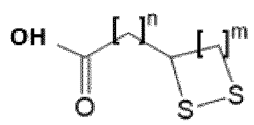

(A')

wherein n is comprised between 0 and 6 and m is comprised between 0 and 5, obtaining polymers with terminal rings having reducible disulfide bridges;
d) optionally adding one or more of the polymers obtained in step a) conjugated with a targeting molecule;
e) partially reducing said polymers obtained in step c) by adding a reducing agent to obtain polymers with free reduced thiol groups, which activate a thiol-disulphide exchange with non-reduced polymers having reducible terminal rings thus forming linear disulphide bonds between the polymers and obtaining a reverse micelle,

wherein at the end of step e) the percentage of thiol groups involved in inter-polymer disulfide bonds with respect to the total of the thiol groups present in the cyclic disulfide groups is comprised between 70 and 100%, preferably between 90 and 100%.

In an embodiment, a further step f) is present wherein a water-soluble pharmaceutical active ingredient is introduced into the obtained reverse micelle by placing a suspension of the reverse micelle obtained at the end of step e) in a non-polar solvent in contact with a solution of the active ingredient in its solution medium.

A reverse micelle obtained by the process above defined is also an object of the invention.

The reverse micelles of the invention are an advantageous vector for molecules generally endowed with poor stability in water solutions or in body fluids. They have indeed been shown to be able to preserve the activity of the delivered molecules. In particular, they allow the passage of macromolecules, such as enzymes, through the blood brain barrier, overcoming the above posed problems. Furthermore, since the micelles of the invention are polymer-based preparations, they can be easily modified by means of appropriate chemical procedures with the aim of adding additional or different functionalities. Also, they can advantageously be obtained by degradable and biocompatible materials.

It is also an object of the invention a polymer of formula (I) as above defined.

It is also an object of the invention a pharmaceutical composition comprising the reverse micelle of the invention loaded with at least one pharmaceutical ingredient and at least one pharmaceutically acceptable vehicle and/or excipient.

## DETAILED DESCRIPTION OF THE INVENTION

**Figures**

**Figure 1.** 1H-NMR spectrum of ene-terminated copolymer.

**Figure 2.** 1H-NMR spectrum of DTT-terminated copolymer.

**Figure 3.** 1H-NMR spectrum of LA2-terminated copolymer.

**Figure 4.** 1H-NMR spectrum of TRIM-terminated copolymer.

**Figure 5.** DLS analysis of RM compared to non-targeted RM.

**Figure 6.** GALC enzymatic activity of RMs measured at different time points after the synthesis (1, 4, 8, 22, 26, and 34 days).

**Figure 7.** Statistics: * = P<0,05; **=P<0,01; Student's t-test or ANOVA (Tukey's multiple-comparisons test). Data are reported as mean ± SEM and each point in the data set represents a single mouse. GALC activity is expressed as nanomoles per hour per milligram of cell lysate and reported in percentage of the WT activity. RMs = reverse micelles, IV = intravenous injection, IP = intraperitoneal injection.

**Figure 8.** Statistics: *=P<0,05; **=P<0,01; ****=P<0,0001 Student's t-test. Data are reported as mean ± SEM and each point in the data set represents a single mouse. GALC activity is expressed as nanomoles per hour per milligram of cell lysate and reported in percentage of the WT activity.

**Figure 9.** Statistics: data are reported as mean ± SEM and each point in the data set represents a single mouse. GALC activity is expressed as nanomoles per hour per milligram of cell lysate and reported in percentage of the WT activity.

**Figure 10.** Statistics: ****=P<0,0001 Student's t-test Data are reported as mean ± SEM and each point in the data set represents a single mouse. GALC activity is expressed as nanomoles per hour per milligram of cell lysate and reported in percentage of the WT activity.

**Figure 11.** Statistics: ***=P<0,001 Student's t-test Data are reported as mean ± SEM and each point in the data set represents a single mouse. GALC activity is expressed as nanomoles per hour per milligram of cell lysate and reported in percentage of the WT activity.

Within the meaning of the present invention, for "reverse micelle" is intended a micelle in which the nonpolar and polar phases have reversed roles with respect to "normal micelle" so that the hydrophilic/polar portions face the inside of the micelle and the hydrophobic/non polar portions face the outside of the micelle.

The micelle of the invention comprises at least two polymers having the formula (i) showed above. In such formula AAA ∿∿ AAA is an hydrophilic polymer which is covalently linked to an hydrophobic polymer, indicated as BBB ∿∿ BBB.

R1, R2 and R3, which can be the same or different from each other, are selected from hydrogen, $C_{1-20}$ alkyl and $C_{1-20}$alkyl-O-$C_{1-20}$alkyl. In particular, they are $C_{1-20}$ alkyl, preferably $C_{1-4}$ alkyl, more preferably they are selected from methyl, ethyl, n-propyl or isopropyl groups. Preferably they are hydrogen or methyl groups.

Y is an alkyl chain, preferably a $C_2$-$C_{10}$ alkyl chain, more preferably a $C_4$ alkyl chain, substituted with at least two molecules of general formula (A):

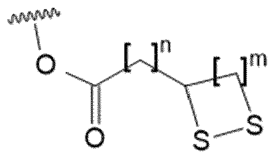

(A)

wherein n is comprised between 0 and 6 and m is comprised between 0 and 5. Preferably, m is 2.

Preferably, Y is substituted with between two and four molecules of formula (A).

Y can be terminally substituted, for example with a thiol group.

In schemes of the present invention, the hydrophilic polymer is indicated as AAA 〰 AAA, wherein the number of A shown is a mere example and not indicative of the number of monomers composing the polymer, which may vary.

The hydrophilic polymer can be selected from the group consisting of: water-soluble poly(ether)s, for example polyethylene glycol; vinyl-based polymers, for example polyvinylpyrrolidone, polyvinyl alcohol; polyacrylamide; acrylic-based polymers, for example poly(acrylic acid) or poly(acrylate)s. All such polymers are known in the field and are commercially available or can be manufactured according to common knowledge in the field.

Preferably, it is polyethylene glycol.

Polyethylene glycol (PEG) is an oligomer or polymer of ethylene oxide. PEG is also known as polyethylene oxide (PEO) or polyoxyethylene (POE), depending on its molecular weight. The structure of PEG is commonly expressed as $H\text{-}(O\text{-}CH_2\text{-}CH_2)_n\text{-}OH$.

PEGs are prepared by polymerization of ethylene oxide and are commercially available over a wide range of molecular weights from 300 g/mol to 10,000,000 g/mol.

In the present invention, PEG preferably has a molecular mass between 500 and 5000 g/mol.

Exemplary PEGs are 2000 Da and 5000 Da.

PEG can be chemically modified, for example by the attachment of one or more chemical groups, such as hydroxyl, carboxyl, thiol or methoxy groups, in the end-chain sites.

A preferred modified PEG is methoxy poly(ethylene glycol).

Modifications can be made as known in the art.

In schemes of the present invention, the hydrophobic polymer is indicated as BBB 〰 BBB, wherein the number of B is a mere example and is not indicative of the number of monomers composing the polymer, which may vary.

The hydrophobic polymer can be selected from the group consisting of: aliphatic polyesters, such as poly(caprolactone), poly(glycolide), poly(l-lactide), poly(lactide-co-glycolide); apolar poly(ether)s, such as polypropyleneoxide; poly(anhydride)s. All such polymers are known in the field and are commercially available or can be manufactured according to common knowledge in the field.

Preferably it is poly (lactide-co-glycolide).

Poly (lactide-co-glycolide) (PLGA) is a copolymer of lactic acid and glycolic acid. Depending on the ratio of lactide to glycolide used for the polymerization, different forms of PLGA can be obtained and they are all suitable for the present invention. PLGA is commercially available or can be manufactured according to common general knowledge in the field.

The hydrophilic polymer and the hydrophobic polymer are covalently linked in the micelle of the invention so as to form a co-polymer, which can herein be named as co-polymer or just as polymer. Such polymer is typically indicated in schemes of the present description as AAA 〰 AAA- BBB 〰 BBB, wherein the number of A and B is a mere example and is not indicative of the number of monomers composing the polymers, which may vary.

A preferred co-polymer is methoxy poly(ethylene glycol)-block-poly(lactide-co-glycolide).

Preferably, the co-polymer has a hydrophilic-lipophilic balance (HLB) between 3 and 6 in the Griffin's scale.

The HLB is calculated as:

$$HLB = 20\frac{M_{hydrophilic}}{M_{overall}}$$

Where $M_{hydrophilic}$ is the molecular weight of the hydrophilic portion and $M_{overall}$ the whole chain molecular weight.

For methoxy poly(ethylene glycol)-block-poly(lactide-co-glycolide) the value of HLB is close to 3 and thus it can be advantageously used in the present invention. This co-polymer is commercially available.

Polymers can be optionally further functionalized, according to the common general knowledge in the field.

In the micelle of the invention polymers of formula (I) are linked to each other through disulfide bonds between the

thiol groups formed upon reduction of the -S-S- linkage in molecule (A).

The percentage of thiol groups involved in inter-polymer disulfide bonds with respect to the total of the thiol groups present in molecules (A) is comprised between 70 and 100%, preferably between 90 and 100%. This means that in some embodiments only a portion of the polymers of formula (I) are linked to each other through disulfide bonds while in other embodiments all polymers of formula (I) are linked to each other through disulfide bonds.

For "inter-polymer disulfide bond" it is intended a disulfide bond between two adjacent polymers of formula (I), in particular between the thiol groups present in molecules (A) of two adjacent polymers of formula (I).

In a preferred embodiment, all the thiol groups of the molecules of formula (A), i.e. 100%, are involved in inter-polymer disulfide bonds.

The following formula (II) represents an example of two exemplary polymers of formula (I) linked by a disulfide bond:

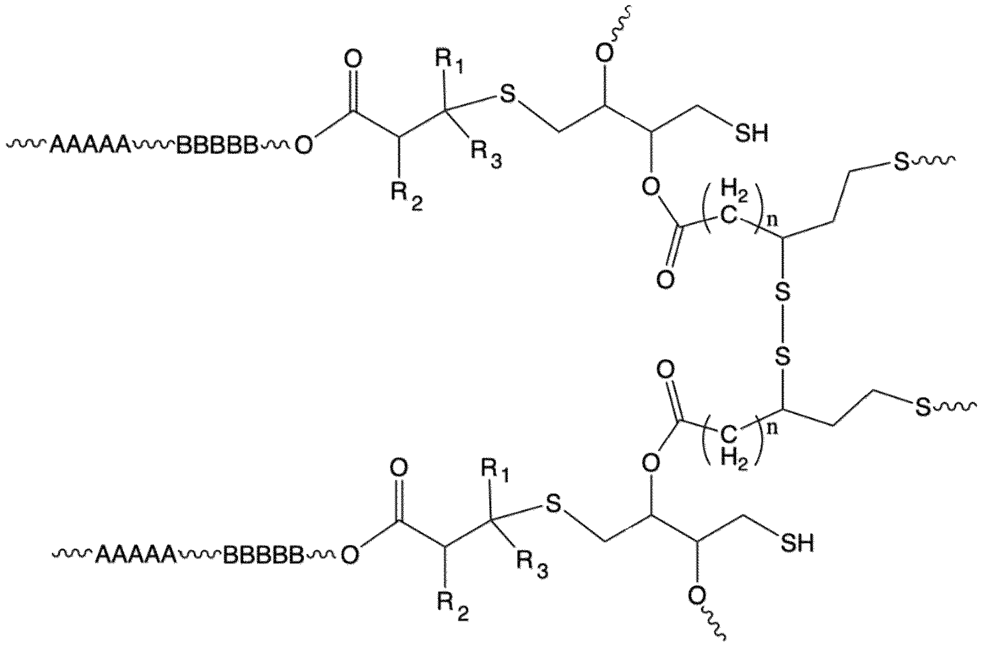

(II)

In a preferred embodiment, the micelle of the invention also comprises polymers of the above formula (III) carrying one or more targeting molecules. In a particular embodiment, the polymers carrying the targeting molecule are a percentage comprised between 0.3 and 5.0% w/w of the total of the polymers forming the micelle.

In formula (III) Z is an alkyl chain, preferably a $C_2$-$C_{10}$ alkyl chain, more preferably a $C_4$ alkyl chain substituted with at least two OH groups. Typically, in a micelle of the invention comprising polymers of formula (I) and (III), AAA, BBB, R1, R2, R3 and x have the same meaning in both polymers.

The conjugation between Z and the targeting molecule is a covalent binding and it can be carried out according to the general knowledge in the field depending on the functional group(s) present on the targeting molecule

For "targeting molecule" it is intended a molecule able to direct the micelle to a determined target, such as a body region or a cellular type or a body tissue. Such targeting molecule can be a peptide; a nucleic acid, such as a DNA or a RNA molecule, in particular oligonucleotides or polynucleotides; a protein; a peptidomimetic or any synthetic functional unit. Targeting molecules are known in the field and can be selected by the skilled person according to the desired therapeutic application of the micelle.

For synthetic functional unit is intended a functional element, such as a peptide or a nucleic acid, of synthetic origin.

Preferably, it is a peptide, for example a peptide selected from Angiopep-2 (having sequence TFFYGGSRGKRNNFKTEEYG; SEQ ID N.1), g7 peptide (GF(D-)TGFLS(O-b-D-glucose); SEQ ID N.2; reference for this peptide can be found in Tosi et al., 2007) and Tf2 peptide (having sequence GGGHKYLRW; SEQ ID N.3).

In a preferred embodiment, it is the Angiopep-2 peptide. Angiopep-2 peptide is a known peptide and commercially available. It can be advantageously used in the micelle of the invention since it is able to cross the blood brain barrier (BBB) and can facilitate the delivery of pharmacological agents to the brain, for example for the treatment of brain tumors.

The reverse micelle of the invention can be obtained by the process described below, which comprises steps

a)-e).

A reverse micelle obtained or obtainable by such process is also an object of the invention.

In step a) a polymer comprising a hydrophilic polymer covalently linked to an hydrophobic polymer, said hydrophobic polymer provided with a terminal hydroxyl group, is modified with the addition of a -ene functional group at the free end-bone, according to the following scheme 1, wherein R1, R2 and R3 are as defined above and X is an halogen, such as fluorine (F), chlorine (Cl), bromine (Br) and iodine (I), preferably it is chlorine:

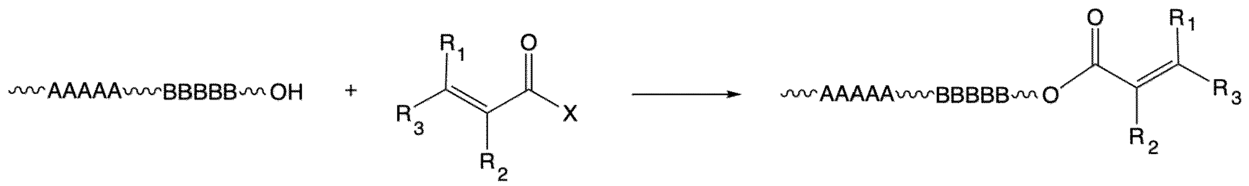

Scheme 1

Hydrophilic and hydrophobic polymers are as disclosed above.

The reaction is typically performed dissolving the co-polymer formed by the hydrophilic and the hydrophilic polymer in a nonpolar solvent together with a nucleophilic catalyst and an alkenyl halide, and maintaining under stirring at room temperature.

The non-polar solvent can be for example chloroform, dichloromethane, benzene, toluene, tetrahydrofuran. Preferably, it is chloroform.

The nucleophilic catalyst can be for example an amine, such as pyridine, N,N-dimethylaminopyridine (DMAP), triethylamine (TEA). Preferably, it is N,N-dimethylaminopyridine (DMAP).

For alkenyl halide is intended a compound whose molecule has one or more halogen atoms bonded to an alkenyl group. The alkenyl group can be a $C_2$-$C_8$ alkenyl, preferably it is a $C_2$-$C_4$ alkenyl. The alkenyl halide can be selected from acryloyl chloride, methacryloyl chloride, crotonoyl chloride, 2-chloroethyl acrylate. Preferably, it is acryloyl chloride.

In step b) the vinyl end-bone of the obtained polymer reacts with the thiol group of an added thiopolyol by thiol-ene click-chemistry.

For thiopolyol it is herein intended a compound having at least one free thiol group and an alkyl chain substituted with at least two hydroxyl groups. Preferably, the thiopolyol comprises two free thiol groups and from two to four hydroxyl groups.

Preferably the thiopolyol is dithiotreitol (DTT).

An exemplary reaction wherein the thiopolyol is DTT is shown in the following Scheme 2.

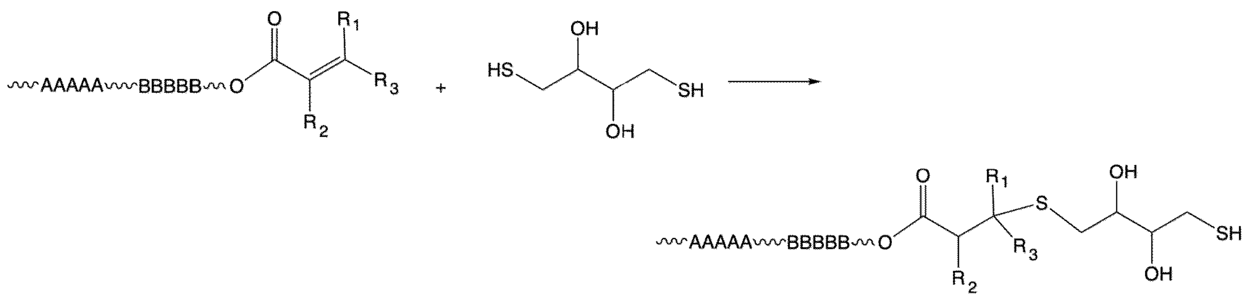

Scheme 2

The functionalization with the thiopolyol endows the macromolecule with at least two hydroxyl functional groups that are subsequently used for the chemical coupling with a cyclic disulfide of formula (A') in step c).

The reaction is performed in a polar or slightly polar solvent. Exemplary polar or slightly polar solvents are acetone, acetonitrile and methyl ethyl ketone, preferably it is acetone. In an embodiment, the reactor is charged with the thiopolyol, such as DTT, dissolved in the selected solvent, then a solution of the polymer in the same solvent is added drop-wise to the reactor and maintained under stirring.

Reaction kinetics can be optionally accelerated by addition of a secondary amine, such as ethylendiamine (EDA), piperidine, pyrrolidine.

In step c) the obtained hydroxyl groups on the polymer are conjugated, for example via EDC (N-(3-Dimethylaminopropyl)-N'-ethylcarbodiimide hydrochloride)/NHS (N-

Hydroxysuccinimide) chemistry, with a cyclic disulfide of formula (A'):

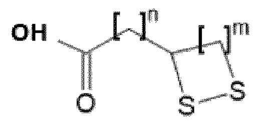

(A')

wherein n is comprised between 0 and 6 and m is comprised between 0 and 5.

Preferably, m is 2.

Preferably, said molecule of formula (A') is lipoic acid.

An exemplary reaction is shown in the following scheme 3:

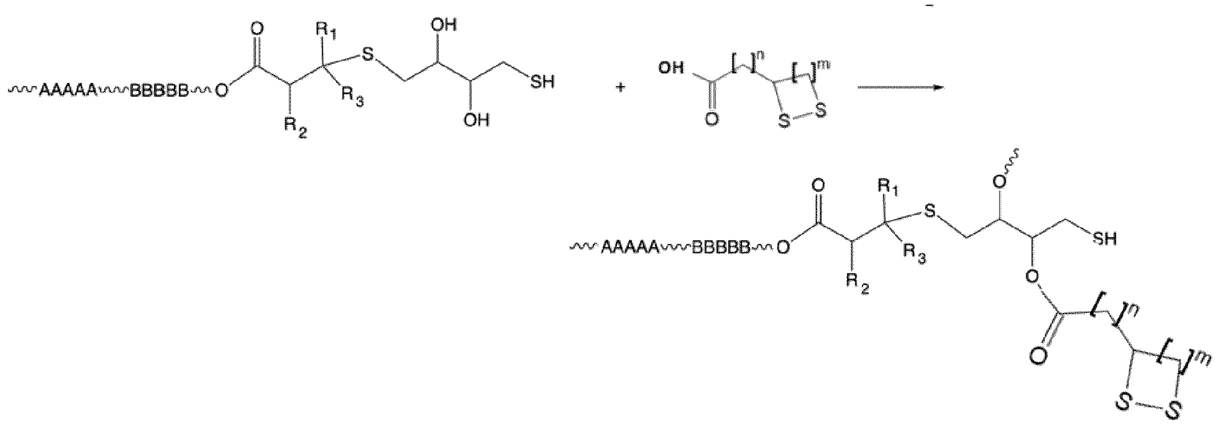

Scheme 3

In an embodiment, the cyclic disulfide (A'), for example lipoic acid, is dissolved in a nonpolar solvent, such as chloroform, together with a nucleophilic catalyst, then a polymer solution in the same solvent is added.

The nucleophilic catalyst can be for example an amine, such as piridine, N,N-dimethylaminopyridine (DMAP), triethylamine (TEA). Preferably, it is N,N-dimethylaminopyridine (DMAP).

In a preferred embodiment, the cyclic disulfide (A'), for example lipoic acid, is added in a large excess in respect to the polymer. Preferably, it is added in an excess amount comprised between 1.5 and 3 with respect to their stoichiometric 1:1 ratio.

In this embodiment, the preparation of the micelle comprises a further optional step d) wherein polymers conjugated to one or more targeting molecules are added.

Said polymer conjugated with the targeting molecule is preferably obtained according to the following steps:

d'. reacting the polymer obtained in step a) of the process with a thiopolyol obtaining a polymer with at least two hydroxyl lateral groups;

d". reacting the obtained polymer with a molecule bearing at least one alkylidene and/or vinyl group;

d"'. conjugating the obtained polymer with a targeting molecule.

The starting polymer is the one obtained in step a) of the process, which can then be modified with a thiopolyol. Modification with a thiopolyol occurs as per step b) described above.

The obtained polymer can then by further modified by reaction with a molecule bearing at least one alkylidene and/or vinyl group.

For alkylidene group it is intended a functional group derived from an alkane by removal of two hydrogen atoms from the same carbon atom, the free valencies being part of a double bond (-R1C(R2)=C(R3)R4).

For vinyl group it is intended a functional group with the formula $-R-CH=CH_2$.

An example of such molecule is trimethylolpropanetrimethacrylate (TRIM).

Finally, the obtained modified polymer can be conjugated with a targeting molecule.

The conjugation between the modified polymer and a targeting molecule can be carried out according to the general knowledge in the field depending on the functional group(s) present on the targeting molecule. In an embodiment the targeting molecule is conjugated to the polymer via a thiol-ene click-chemistry.

In a preferred embodiment, the polymer is functionalized with DTT, dissolved in a suitable nonpolar solvent, such as chloroform, then trimethylolpropanetrimethacrylate (TRIM) and a nucleophilic catalyst are added and a TRIM-terminated polymer is obtained.

The nucleophilic catalyst can be for example an amine, such as piridine, N,N-dimethylaminopyridine (DMAP), triethylamine (TEA). Preferably, it is triethylamine (TEA).

An exemplary formation of a TRIM-terminated polymer is shown in the following scheme 4:

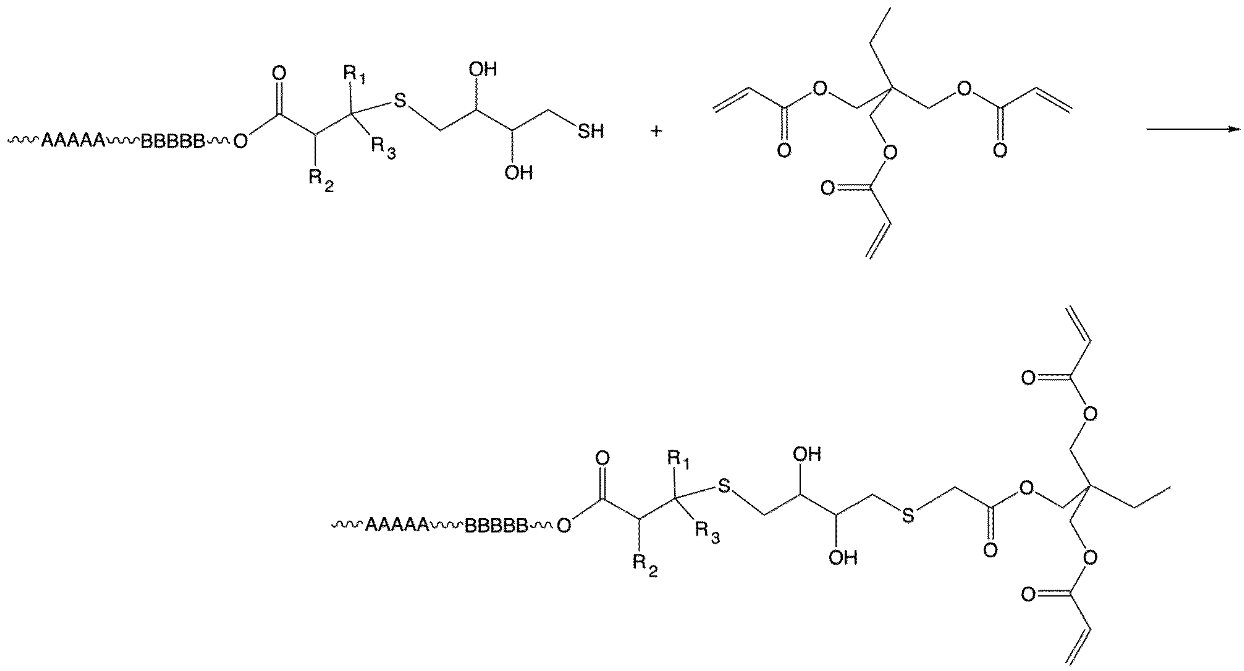

Scheme 4

The obtained TRIM-terminated polymer is then used for the conjugation with the targeting molecule.

For example, TRIM-terminated polymer is dissolved in a slightly polar or polar solvent, such as acetone. Once the TRIM-terminated polymer is dissolved, the targeting molecule dissolved in a suitable solvent is added and the polymer conjugated with the targeting molecule is obtained.

In step e) partial reduction of the thiol groups present on the terminal rings attached to the modified polymer occurs, i.e. terminal rings are opened to yield dihydro groups.

For example, when the polymer has been functionalized with lipoic acid, lipoic rings are opened to yield dihydrolipoyl groups as per the following Scheme 5:

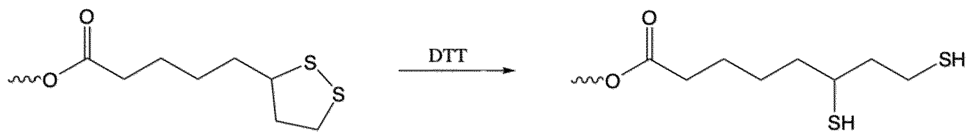


Scheme 5 - Formation of hydrolipoyl groups.

Reduction is performed by addition of a reducing agent, for example DTT.

The reducing agent can be any suitable reducing agent as known in the art. For example it can be selected from cysteine hydrochloride, 2-mercaptoethanol, dithiothreitol, dithiobutylamine, glutathione reduced, tris(2-carboxyethyl)phosphine, ascorbic acid and sodium sulfite.

For partial reduction is intended that only a portion of the terminal rings of the polymers obtained at the end of step c) is reduced, while a portion of the polymers maintain oxidized terminal rings. The portion of reduced polymers may be for example between 30 and 50% of the total of the polymers obtained.

The partial reduction is modulated by the concentration of the reducing agent used in step e). Concentration of reducing agent is lower with respect to the concentration which would be needed to obtain a total reduction of the disulphide bonds present on the rings of the polymers. In an embodiment, the concentration of the reducing agent, such as DTT, is between 30 and 50% of cyclic sulfide moles.

The obtained polymers with reduced dihydro groups activate a thiol-disulphide exchange when interacting with other thiol terminal rings present on non-reduced polymers, forming linear disulphide bonds between the polymers.

For thiol-disulfide exchange is intended a process in which the free thiol attacks the disulfide, breaking the -S-S- bond, with subsequent formation of a new disulfide between the previously free thiol and the thiol of the original disulfide compound.

The following scheme 6 illustrates an exemplary formation of disulfide bonds between the polymers:

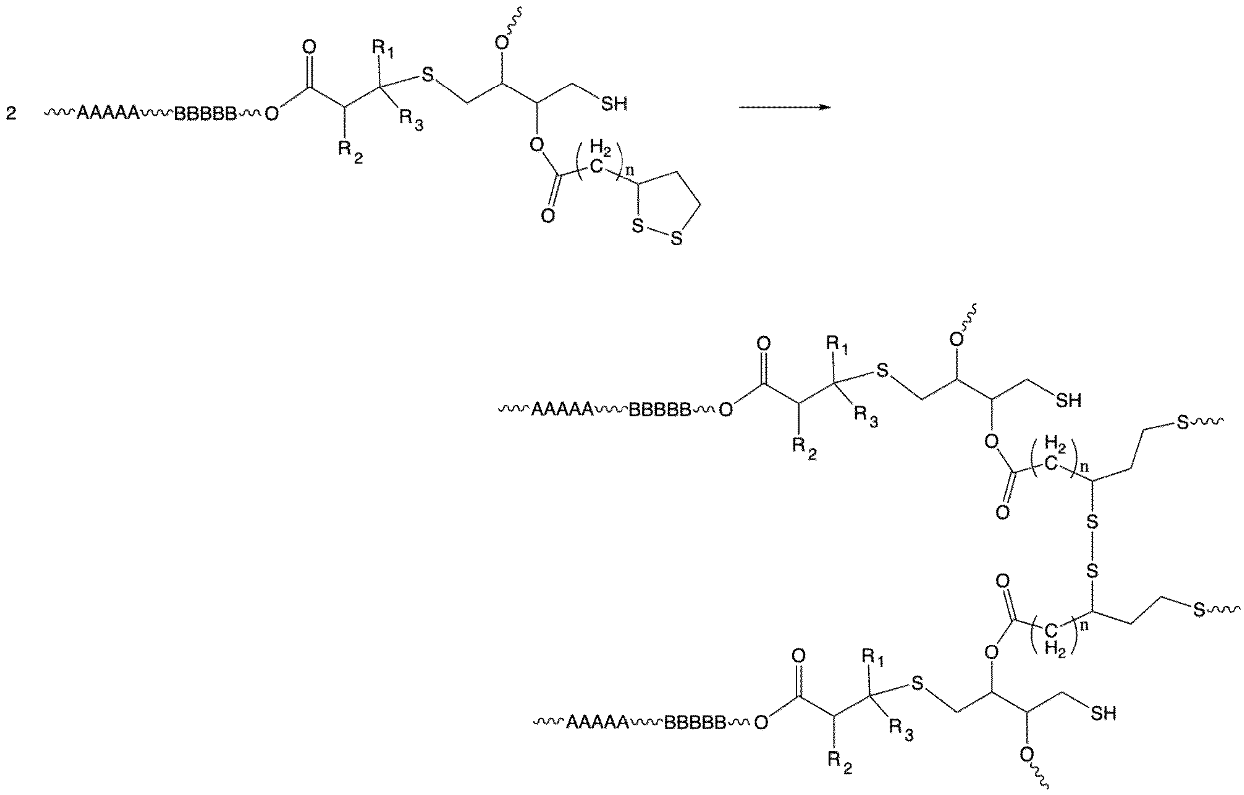

Scheme 6

An embodiment wherein the cyclic disulfide is lipoic acid is illustrated in the following scheme 7, wherein the lipoic acids are intended to be attached to the polymer (not shown):

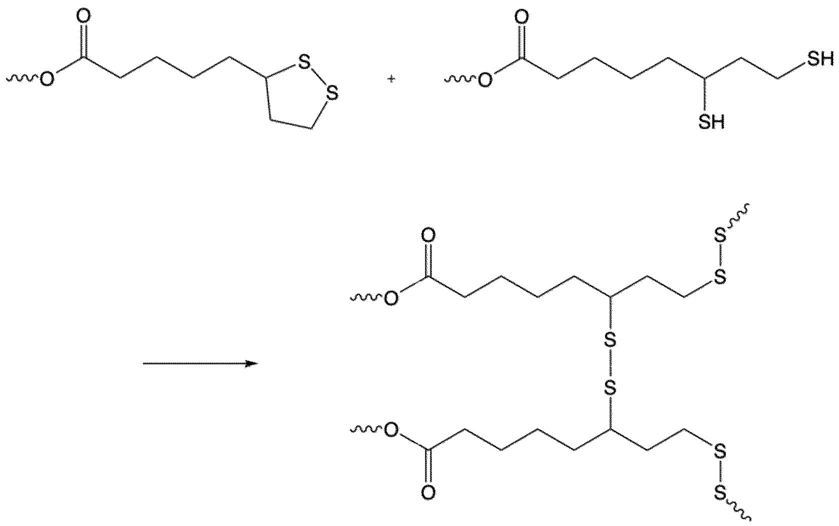

Scheme 7 – Formation of linear disulphide bonds after the thiol-disulphide exchange between dihydrolipoyl groups and closed lipoic acid rings.

Preferably, the reaction continues until all proximal thiol rings are crosslinked with at least one disulphide bond.

This reaction brings to the formation of a reverse micelle where all or most of adjacent polymers are cross-linked one to the other through disulfide bonds.

Optionally, distilled water is added to the mixture in step e) before addition of the reducing agent to trigger the micellization process.

At the end of the reaction of step e) a reverse micelle according to the invention is obtained.

The reverse micelle of the invention can be further modified according to the general knowledge in the field depending on the desired application. For example further functionalities can be added on the polymers or a coating can be applied.

The reverse micelle of the invention can be loaded with a water-soluble active pharmaceutical ingredient.

Said pharmaceutical ingredient is preferably a macromolecule. For macromolecule it is intended a molecule with dimensions in the range 3,000-150,000 Da.

Said pharmaceutical ingredient can be any pharmaceutical ingredient. For example, it can be an active pharmaceutical ingredient for the treatment of at least one disorder or condition selected from the following group: leukodystrophies, diseases of the central nervous system, such as neurodegenerative diseases, and lysosomal storage disorders.

In a preferred embodiment, it is a pharmaceutical ingredient which is for the treatment of a disorder of the central nervous system, such as a brain tumor, Alzheimer disease or Parkinson disease, and which would therefore benefit of the drug delivery system of the invention which is able to cross the blood brain barrier.

Said pharmaceutical ingredient can be selected from:

- a protein, in particular a recombinant protein or a cytokine, for example DNL310, Filgrastim, JR-141, JR-171, Migalastat or NKTR-214;
- a peptide, for example GV1001, rh-Endostatine or Sargramostim;
- an enzyme, for example Avalglucosidase, Cipaglucosidase Alfa, Elosulfase, Galsulfase, Iduronidase, Idursulfase, Imiglucerase, Laronidase, N-acetylgalactosamine 6-sulfatase, Taliglucerase Alfa, Velaglucerase alfa, Vestronidase alfa, $\alpha$-galactosidase A (a-GAL), $\alpha$-glucosidase (GAA), $\alpha$-N-acetylglucosaminidase, $\beta$-Glucocerebrosidase (GCase), galactosylceramidase (GALC);
- a nucleic acid, such as those used in gene therapy, for example ABO-101, ABO-102, AT845, AVR-RD-02, AXO-AAV-GM2, FBX-101, OTL-200, PBKR03, PR001, RGX-111, RGX-121, SPK-3006 or TSHA-101;
- an antibody, such as Adalimumab, Bepranemab, Bevacizumab, Camrelizumab, Cetuximab, Durvalumab, Ipilimumab, m266, Magrolimab, Natalizumab, Nivolumab, Omburtamab, Panitumumab, Pembrolizumab, Pepinemab, Pritumumab, Rituximab, Sotigalimab, TB006 or Trastuzumab.

Preferably, the active ingredient is introduced into the reverse micelle of the invention by placing a suspension of the reverse micelle in a non-polar solvent, such as chloroform, in contact with a solution of the active ingredient in its

solution medium, for example a buffer or physiological solution. The reverse micelles of the invention are able to bring the hydrophilic molecules into solution in the nonpolar phase. Advantageously, any phenomena of precipitation of the active principle at the interface with the nonpolar solvent resolves spontaneously, as the aqueous phase depletes of the active ingredient which is captured by the micelles.

The reverse micelle of the invention loaded with a pharmaceutical ingredient can be administered by any conventional administration method. Preferably it is administered by parenteral or intravenous injection, but other forms are equally suitable. Methods for preparing administrable (e.g., parenterally administrable) compositions are known or apparent to those skilled in the art and are described in more detail in, for example, Remington's Pharmaceutical Science (17th ed., Mack Publishing Company, Easton, PA, 1985).

The person skilled in the art will decide the suitable administration method and the effective timing of administration, depending on the patient's conditions, degree of severity of the disease, response of the patient and any other clinical parameter within the general knowledge of this matter.

The reverse micelle of the invention loaded with a pharmaceutical ingredient can be included in a pharmaceutical composition which may further include at least one pharmaceutically acceptable vehicle and/or excipient. These may be particularly useful formulation coadjuvants, e.g. solubilising agents, dispersing agents, suspension agents, and emulsifying agents, buffering agents and/or preservatives, as known in the art.

Average quantities of the active ingredient may vary and in particular should be based upon the recommendations and prescription of a qualified physician.

The reverse micelle of the invention loaded with a pharmaceutical ingredient can be advantageously used for the treatment of a disease which can be treated by the loaded pharmaceutical ingredient. In particular, thanks to its ability to deliver macromolecules, the micelle can be advantageously used for enzyme replacement therapy, i.e. to deliver a deficient enzyme. For example, the reverse micelle can be loaded with the enzyme galactosylceramidase (GALC) and be used for the treatment of Krabbe Disease (KD, or Globoid Cell Leukodistrophy), which is characterized by a deficiency of GALC.

The invention will be now described by means of illustrative examples.

## EXAMPLES

### Example 1

### GALC-loaded stabilized reverse micelles preparation and characterization

### Materials and methods

*Selection of polymer composition and molecular weight*

In order to improve the micellization process, the starting point is the identification of a material with a suitable hydrophilic-lipophilic balance (HLB) to cover the range between 3 and 6 in the Griffin's scale.

The HLB is calculated as:

$$HLB = 20\frac{M_{hydrophilic}}{M_{overall}}$$

Where $M_{hydrophilic}$ is the molecular weight of the hydrophilic portion and $M_{overall}$ the whole chain molecular weight.

The polymer selected for this work is the methoxy poly(ethylene glycol)-block-poly(lactide-co-glycolide) with $M_{hydrophilic}$ = 2 kDa and $M_{overall}$ = 13.5 kDa. For this polymer, the value of HLB is close to 3 and thus it is suitable for the proposed application.

*Control of reverse micelle size*

The preparation of reverse micelles (RMs) is governed by the molar ratio of water-to-surfactant WO, given by [Tang et al., 2018]:

$$W_0 = \frac{[H_2O]}{[S]}$$

In this equation, [$H_2O$] and [S] are the molar concentrations of water and surfactant (polymer) respectively.

RMs prepared from surfactants with a low molecular weight are aggregates containing a small amount of water, with a WO less than 15, whereas droplets containing a large amount of water molecules WO > 15 are regarded as microemulsions [Pileni et al., 1993]. The curvature for reverse micelles corresponds to the energetically favorable packing configuration of surfactant molecules at the interface [Kotlarchyk et I., 19985; Safran et al., 1983] and the micellar radius related with moles of surfactant and water per micelle.

*Reverse micelle stabilization*

Starting from preparation and *in vitro* modification to final applications (e.g. iv injection), micelles undergo some environmental changes (like formulation concentration, freeze-drying, dilution in the injection medium, dilution upon in vivo injection, presence of salts or blood proteins, pH changes, and so on). For drug delivery applications, micelles have to remain intact to preserve their drug cargo until the biological target is reached.

Both thermodynamic and kinetic aspects related to stability should be considered to prevent micelle disintegration and premature delivery of drug cargo [Owen et al., 2012]. The thermodynamic stability of micelles describes all phenomena leading to micelle formation and their equilibrium. The kinetic stability is related to their behaviour over time, including exchange and assembly/disassembly phenomena.

Since RM should undergo additional processes after the preparation (e.g. coating, targeting conjugation) in polar environments, their structure should be stable enough to maintain the reverse configuration, without switching to a direct configuration, or lost/share molecules with other micelles or with the coating layer. A correct approach would be the mild stabilization of bare RMs through the crosslinking of external macromolecular end-bones. A biocompatible and mild crosslinking can be obtained through the formation of intermolecular -S-S- bridges, that can be easily degraded in the in vivo environment. Micelle stabilization needs of some chemical modifications on the PLGA block end-bone, in order to add specific functional groups providing crosslinkable sites.

The first step is the insertion of vinyl groups reacting the terminal -OH functionality of PLGA with acryloyl chloride (Step 1); the second step is the thiol-ene click chemistry between vinyl groups and dithiotreitol to add two -OH lateral functionalities (Step 2); the third step is the conjugation of lateral -OH with lipoic acid providing rings with oxidable disulphur bridges (Step 3). The last step is the oxidation of lipoic acid rings to give dihydrolipoyl reduced groups that exchange with other non-oxidized lipoic acid units to obtain the intermolecular disulphur bridges (Step 4).

**Step 1: mPEG-block-PLGA-=**

The first step is the modification of the commercial copolymer with the addition of a -ene functional group at the free PLGA end-bone (Scheme 8). This reactive group is needed for the second step of functionalization.

The reaction is performed dissolving the copolymer in a nonpolar solvent (chloroform) together with N,N-dimethylaminopyridine (DMAP) and acryloyl chloride, maintaining under stirring at room temperature.

20 mL of chloroform and 500 mg of mPEG-block-PLGA (molecular weight 13.5 kDa) are added to a one-neck round-bottom glass reactor and stirred at room temperature. Once the polymer is dissolved, 6.4 mg of 4-dimethylaminopyridine (DMAP) and 5.1 μL of methacryloyl chloride (MAC) are added. The reaction is maintained under stirring for 24h at room temperature. At the end of the reaction, the product is precipitated in 70 mL of diethylether, dried and stored at 4°C before the use.

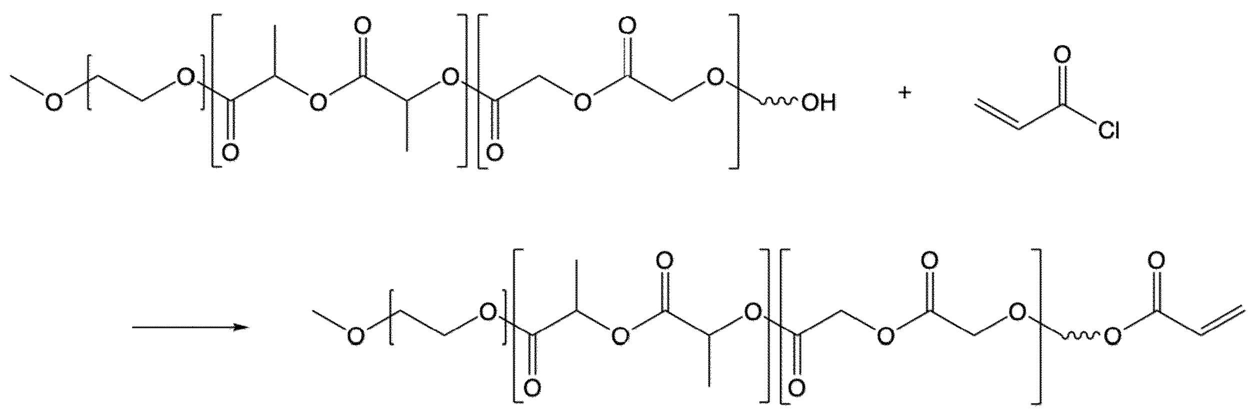

Scheme 8 – Reaction scheme of ene-terminated copolymer

**Step 2: mPEG-block-PLGA-DTT**

The vinyl end-bone reacts with the thiol group of the dithiotreitol (DTT) by thiol-ene click-chemistry to give a thiol-terminated copolymer (Scheme 9). The functionalization with DTT endows the macromolecule with two hydroxyl functional groups that are following used for the chemical coupling with lipoic acid in the third step of functionalization.

The reaction is performed in a polar or slightly polar solvent (acetone). The reactor is charged with DTT dissolved in the selected solvent, then a solution of the copolymer in the same solvent is added drop-wise to the reactor and maintained under stirring. Reaction kinetics accelerates by adding a secondary amine (ethylendiamine, EDA).

8 mg of DTT are dissolved in 1 mL of acetone and 5 μL of EDA, then 200 mg of polymer, dissolved in 10 mL of acetone, are added dropwise. After 24 the product is precipitated in diethyl ether and stored at 4°C before the use.

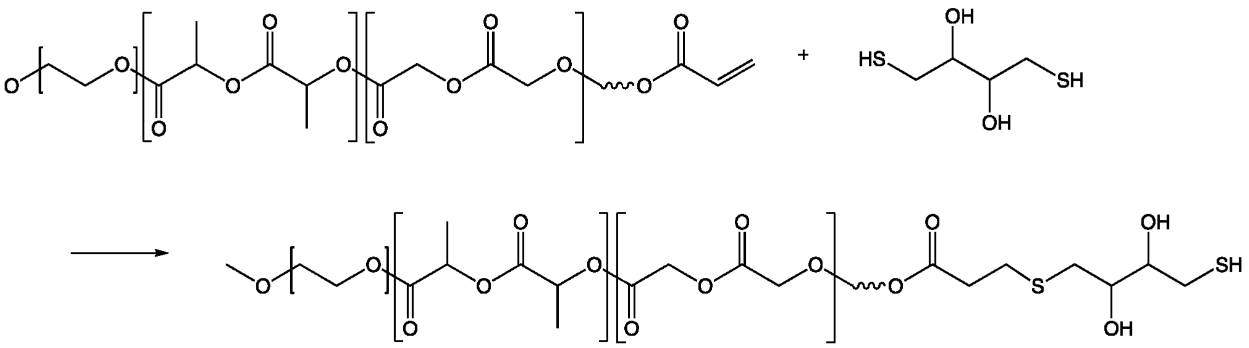

Scheme 9 – Reaction scheme of DTT-terminated copolymer

**Step 3: mPEG-block-PLGA-LA2**

The last functionalization consists in the coupling of -OH functionalities with two molecules of lipoic acid. In this reaction, hydroxyl groups given by the DTT are conjugated via EDC/NHS chemistry with lipoic acid (Scheme 10). The lipoic acid is dissolved in a nonpolar solvent (chloroform) together with DMAP, then a copolymer solution in the same solvent is added. The lipoic acid should be in a large excess in respect to the copolymer.

85 mg of DTT-terminated polymer are dissolved in 10 mL of chloroform. Once the polymer is dissolved, 1.1 mg of DMAP are added and mixed for 5 min. Then, 15.2 mg of lipoic acid (LA) are added. The reaction is maintained at room temperature for 24 h, then the material is dried and washed with ethanol three times to eliminate DMAP and residual LA. The product is dried under vacuum and maintained in a glass vial before use.

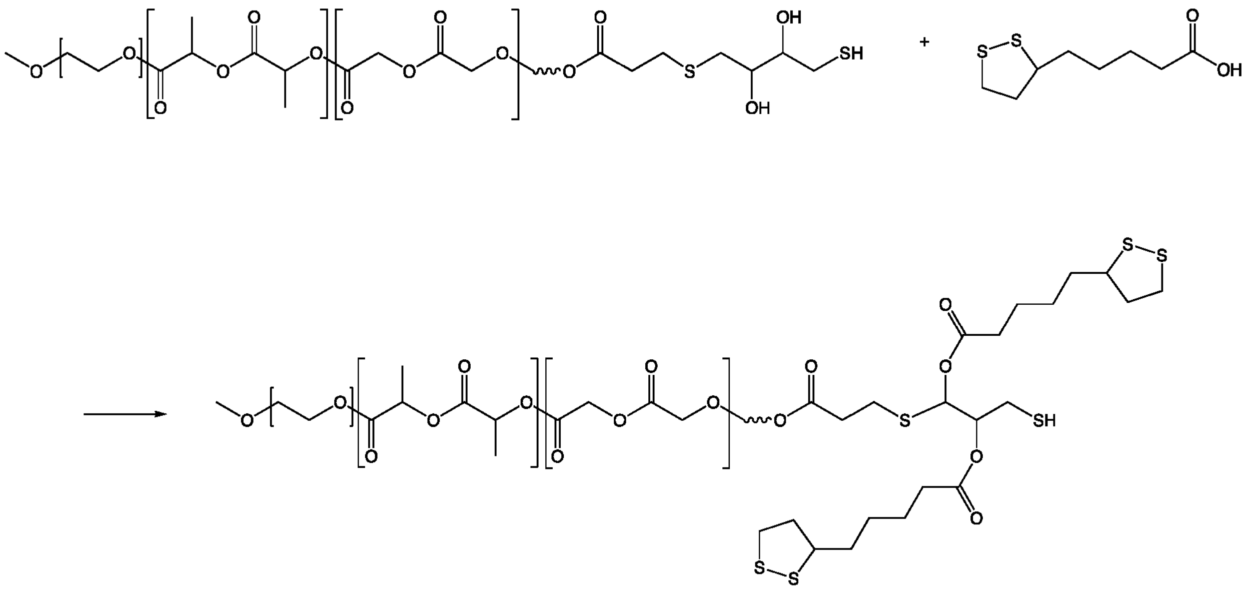

Scheme 10 – Reaction scheme of LA-terminated copolymer

*Synthesis of fluorescent PLGA*

The mPEG-block-PLGA copolymer is marked with a fluorescent dye for confocal microscopy analysis. This is a three-step functionalization. The first step consists in the functionalization of the -OH terminated commercial copolymer with succinic anhydride, in order to obtain a - COOH terminated copolymer.

100 mg of OH-terminated polymer are dissolved in 20 mL of chloroform. Once the polymer is dissolved, 10.9 mg of DMAP are added. The solution is stirred for a few minutes, then 7.4 mg of succinic anhydride and 5 μL of TEA are added. The reaction is maintained under stirring for 24 h, then the solvent is evaporated in rotavapor and the dry polymer is washed twice with methanol. Finally, the material is dried and stored at 4°C in a closed glass vial.

The second step consists in the derivatization with the active ester NHS.

100 mg of COOH-terminated polymer are dissolved in 20 mL of chloroform. Once the polymer is dissolved, 14.2 mg of EDCI are added. The solution is stirred for a few minutes, then 8.5 mg of NHS and 5 μL of TEA are added. The reaction is maintained under stirring for 4 h, then the solvent is evaporated in rotavapor and the dry polymer is washed twice with methanol. Finally, the material is dried and stored at 4°C in a closed glass vial.

The third step is the conjugation of the fluorophore. In our case, the ATTO633-NH2 is selected.

50 mg of NHS-terminated polymer are dissolved in 20 mL of chloroform. Once the polymer is dissolved, 200 μg of ATTO633-NH2 are added. The reaction is maintained under stirring for 18 h, then the solvent is evaporated in rotavapor and the dry polymer is washed twice with methanol. Finally, the material is dried and stored at 4°C in a closed glass vial.

*Synthesis of the Ang-2-conjugated copolymer*

We selected the Ang-2 peptide as targeting unit. This peptide is conjugated to the copolymer via a thiol-ene click-chemistry.

The first step is the synthesis of the TRIM-terminated copolymer.

100 mg of DTT-terminated polymer are dissolved in 20 mL of chloroform. Once the polymer is dissolved, 47.3 μL of trimethylolpropanetrimethacrylate (TRIM) and 5 μL of TEA are added. The reaction is maintained under stirring for 24 h, then the solvent is evaporated in rotavapor and the dry polymer is washed twice with methanol. Finally, the material is dried and stored at 4°C in a closed glass vial.

The TRIM-terminated copolymer is used for Angiopep-2 conjugation.

50 mg of TRIM-terminated polymer are dissolved in 10 mL of acetone. Once the polymer is dissolved, 21.9 mg of Ang-2 dissolved in acetone (1 mg/mL) is added. The reaction is maintained under stirring for 48 h, then the polymer is precipitated in methanol, dried and stored at 4°C in a closed glass vial before the use.

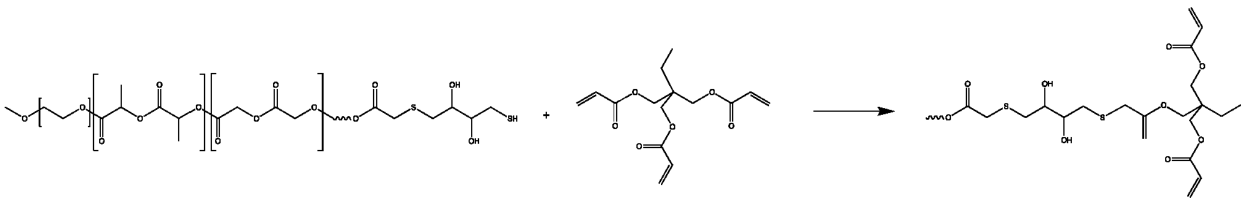

Scheme 11 – Reaction scheme of TRIM-terminated copolymer

*Reverse micelle crosslinking*

Micelles are prepared by using the modified copolymer and, at the end of the micellization procedure, -S-S-bonds are crosslinked by using a catalytic amount of DTT.

Crosslinking reactions occur only on the terminal ring of the lipoic acid but would be strong enough to stabilize SRMs also in polar environment.

The chemical mechanism starts with the activation of lipoic rings by means of DTT. Under the catalysis of DTT, lipoic rings are opened to yield dihydrolipoyl groups (Figure 5).

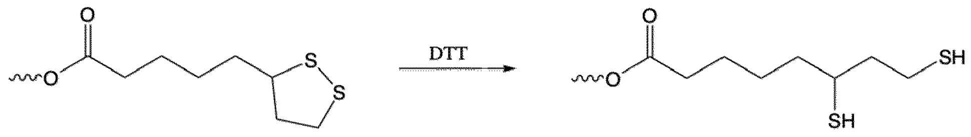


Scheme 12 - Formation of hydrolipoyl groups.

Dihydrolipoyl groups activate a thiol-disulphide exchange when interacting with other lipoic acid rings forming linear disulphide bonds (Scheme 13).

This reaction repeats consequently until all proximal dihydrolipoyl/lipoic acid rings are crosslinked.

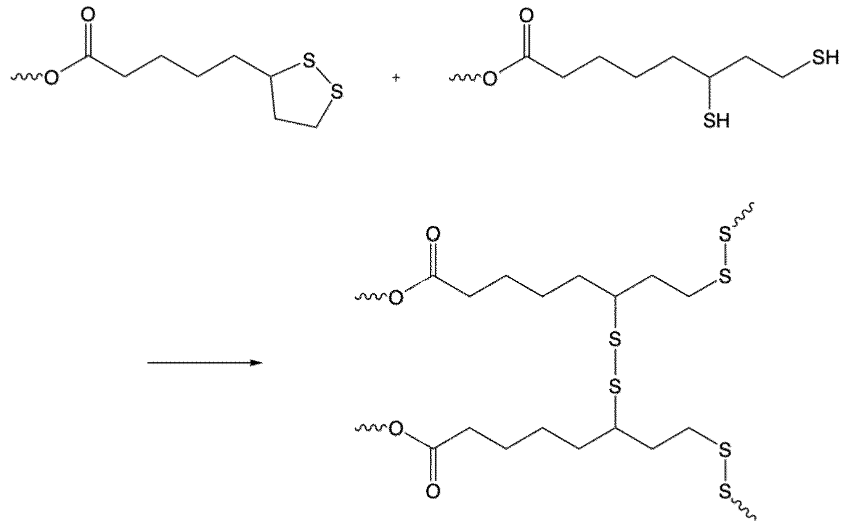

Scheme 13 – Formation of linear disulphide bonds after the thiol-disulphide exchange between dihydrolipoyl groups and closed lipoic acid rings.

18 mg of LA2-terminated polymer are dissolved in 20 mL of chloroform. 1 mg of the Ang2-conjugated or TRIM-terminated copolymer (respectively, for the preparation of targeted and non-targeted micelles) and 1 mg of the ATTO633-terminated copolymer, both dissolved in chloroform, are added. Then, 0.68 mL of distilled water are added to the mixture to trigger the micellization process. Water is added in four aliquots of 0.17 mL, at 5 min per time. After 1.5 h, 0.8 mg of DTT dissolved in chloroform are added. The mixture is flushed with dry N2, sealed and maintained at room temperature and without stirring for 24 h. At the end of the reaction, RM dispersion is dried with rotavapor, resuspended in ethanol and dialyzed over ethanol to remove residual DTT and chloroform. After dialysis, micelles are dried again and stored at 4°C before enzyme loading.

Due to the apparent toxicity of RM, the amount of ANG2-conjugated polymer is reduced to 1/30, 1/40 and 1/50 of the starting recipe, while the overall amount of polymer is maintained.

*GALC encapsulation and enzymatic activity*

GALC encapsulation in RM is obtained maintaining RM dispersed in chloroform in contact with a GALC solution in its buffer (concentration 2.2 mg/ml). 15 mg of crosslinked micelles are dispersed in 0.8 ml of DMC, then 0.56 ml of GALC solution, corresponding to 1.23 mg of enzyme, are added. The mixture is maintained at 4°C for 48 h under mild stirring, then the non-miscible top layer (residual GALC + buffer) is removed, and loaded RMs are dried under vacuum for 3 h. Once dried, RMs are stored in a plastic vial at -20°C before the use. To determine GALC activity in RM samples, 25 μl of RMs was added to 25 μl of 1 mM 4-methylumbelliferyl-β- D-galactopyranoside diluted in assay buffer (50 mM sodium citrate, 125 mM NaCl, and 0.5% Triton X-100 (pH 4.5). Samples were incubated at 37 °C for 2 hours, and 150 μl of stop solution (0.5 M glycine, 0.3M NaOH) was added. 100 μl of samples were plated into a 96-well multiplate for fluorescence, and fluorescence was measured with the Promega GloMax discover Multimode microplate reader with an excitation filter of 365 nm and an emission filter of 415 to 485 nm.

*NMR analysis*

Polymer functionalization is verified by Nuclear Magnetic Resonance. Polymer samples are dissolved in deuterated chloroform or deuterated dimethylsulphoxide (only the ANG2-terminated polymer) at the concentration of 20 mg/mL and analyzed. 1H-, 13C- and DEPT135 spectra are collected.

*DLS analysis*

RM diameter and polydispersion is evaluated with Dynamic Light Scattering (DLS) analysis. 50 μL of sample (50 mg/mL) are dispersed in 200 μL of distilled water. Diameters are evaluated at 90° with a 633 laser.

**2. Results**

**Chemical functionalization of polymers**

End-chain functionalizations are verified with NMR analysis. 1H-NMR spectra are reported in figures 1-4.

Figure 1: 1H-NMR (300 MHz, CDCl3): δ / ppm 5.2 (-OCH(CH3)COO-), 4.8 (-OCH2COO-), 4.2-4.5 (-CH2OCHO-), 1.6 (- OCH(CH3)COO-); for acryloyl end-functionalities, signals of geminal protons overlap with signals in the range 4.5-4.9 ppm, while the signal due to the COOCH=CH2 proton appeared at 6.7ppm.

Figure 2: Additional signals at 3.7 ppm (-CHOH-), 3.2 ppm (-CH2SH), 2.7 ppm (-CHH-), 1.6 ppm (-CHH-) attributed to DTT.

Figure 3: Additional signals at 3.3 ppm attributed to lipoic ring.

Figure 4: Additional signals at 6.1 ppm and 5.5 ppm, attributed to geminal protons of vinyls groups, and 4.1 ppm, attributed to methylol protons.

**DLS analysis**

Diameter evaluation highlights the effect of ANG2 conjugation on RM size, which significantly increases from 98.7 nm to 135.3 nm (Figure 5).

**GALC enzymatic activity of RM over time**

As we can see from figure 6, the GALC enzymatic activity of RMs is maintained up to 34 days after the synthesis.

**Example 2**

**Reverse micelles - mediated enzymatic activity recovery in Twitcher mice**

**Materials and methods**

*Animal procedures*

TWI heterozygous mice (TWI+/- C57BL6 mice; Jackson Labs), kindly donated by Dr. A. Biffi (San Raffaele Telethon Institute for Gene Therapy, Milan, Italy), were used as breeder pairs to generate homozygous TWI mice (TWI-/-, elsewhere abbreviated as TWI for simplicity). TWI animals were intravenously (retro-orbital) injected with RMs or with free GALC diluted in a maximum of 200 μl of physiological solution. After 4 or 24 hours, mice were deeply anesthetized with a urethane solution and sacrificed by transcardial perfusion with phosphate buffer saline (PBS). Subsequently, brain, sciatic nerves, liver and kidneys were extracted from each mouse and stored in the RIPA buffer for the subsequent lysis. Then, organs were immediately processed to perform the GALC enzymatic activity assay and the protein quantification assay. For selected experiments, one liver lobe was placed in 4% PFA solution and stored at 4°C for a minimum of 3 days before vibratome cutting. GALC enzymatic assay has been performed using 6-hexadecanoylamino-4-methylumbelliferyl-beta-D-galactopyranoside (HMU-βGal), the fluorescent substrate currently used for the clinical diagnosis of Krabbe disease. Briefly: 10 μl of lysates were added to 20 μl of 50 μM HMU-betaGal substrate solution and incubated for 17 h at 37°C; the reaction was then stopped and the fluorescent product 4-methylumbelliferone (4-MU) was read by using a microplate GloMax fluorescence reader. GALC activity (as nmol/mg protein extract in 17 h of incubation) was calculated by comparison with a standard curve previously obtained by measuring the fluorescence of different concentrations of 4-MU. For the quantification of cell lysates, a micro-bicinchoninic acid (BCA) protein assay kit has been used.

*Tissues confocal imaging*

For tissue imaging, the liver samples were processed as described in the animal procedure paragraph. Samples were cut with a vibratome (Leica VT1000 S) at a final slice thickness of 60 μm. All samples were mounted with Fluoroshield mounting medium with 4',6-diamidino-2-phenylindole. Samples were imaged with a Leica TCS SP5 SMD inverted confocal microscope (LeicaMicrosystems AG) interfaced with Ar, diode-pumped solid-state (DPSS), and HeNe lasers for excitation at 488, 560, and 633 nm, respectively, and with an external pulse diode laser for excitation at 405 nm. Samples were viewed with a 40× 1.5 numerical aperture oil immersion objective (Leica Microsystems with pinhole aperture set at 1.0 Airy). All images were analyzed with Fiji software.

*Statistical analysis*

Data are reported as mean ± SEM and were statistically analysed by using Prism 6.00 (GraphPad Software, San Diego, CA; RRID:SCR_002798). For parametric data, Student's t-test or one-way ANOVA was used; the mean values obtained in each repeated experiment were assumed to be normally distributed about the true mean. Statistical significance refers to results for which $P < 0.05$ was obtained.

**Results**

**First enzyme replacement therapy (ERT) tests**

The first in-vivo ERT experiments have been done with formulations F1 [enzymatic activity (E.A.) = 30.1 nmol/h/mg] and F2 (E.A. = 322.8 nmol/h/mg).

Despite the increasing E.A. of F2 we could not find E.A. recovery in the brain of TWI treated mice (see Figure 7, brain graph). E.A. increased, instead, in the other tested organs (see Figure 7: sciatic nerve, liver and kidney). Subsequently, we tested F3 (modified PLGA that binds more Ang2, see example 1 above). With this formulation, E.A. increased also in the brain of treated TWI mice, but mice died after 30 minutes from the injection, demonstrating high F3 toxicity.

Toxicity tests were performed and a safe formulation of the RMs containing the modified PLGA, the Ang2 peptide and the enzyme GALC at a concentration of 5 mg/ml in a volume of 50 ul was identified. Thus, we proceeded with this safe combination for the subsequent in-vivo ERT experiments.

**Enzyme replacement therapy tests with the not toxic parameter combination**

The brain of the TWI mice treated with the not toxic formulation (F6; 5 mg/ml; 50 ul) showed an E.A. significantly higher than the E.A. of the TWI untreated (see Figure 8: $P < .001$ HOM RMS 4H vs. HOM NT; Student's t-test) 4 hours after the RMs administration. Additionally the activity is maintained also after 24 hours from the injection ($P < .0001$ HOM RMS 4H vs. HOM NT; Student's t-test).

Overall, these data demonstrate that the F6 formulation gives to the TWI brain an E.A. that is maintained to a level that could be potentially of clinical interest (≈ 15 % of TWI untreated) up to 24 hours from the treatment.

Regarding the other organ of the nervous system that we analyzed, the sciatic nerve, we found different results in respect to the brain (Figure 9). The E.A. of TWI treated mice was not significantly increased after 4 and 24 hours. It is known that efficient drug delivery of therapeutic doses to this district can be difficult to achieve, owing to neuroanatomy issues and the restrictiveness of the blood-nerve barrier.

We also tested the E.A. in two typical accumulation organs: liver (figure 10) and kidneys (figure 11). Regarding the liver, we found a high E.A. in the liver of TWI mice treated with F6 after 4 hours from the treatment (see the graph in Figure 10: $P < .0001$ HOM RMS 4h vs. HOM NT), confirming the capability of our RMs to deliver enzymatically active GALC into mice tissues. As expected, 24 hours from the treatment the E.A. decreased, due to the physiological liver disposal mechanisms. We confirmed the presence of RMs in the liver of the treated animal also by confocal microscopy.

Regarding the kidneys, we found data similar to the liver (Figure 11). E.A. after 4 hours is significantly increased in respect to untreated TWI mice, whereas it decreased after 24 hours, likely due to the physiological kidneys excretion processes.

**References**

Koyamatsu, Y., Hirano, T., Kakizawa, Y., Okano, F., Takarada, T., & Maeda, M. (2014). pH-responsive release of proteins from biocompatible and biodegradable reverse polymer micelles. Journal of Controlled Release, 173, 89-95.

Xu, Y., Meng, F., Cheng, R., & Zhong, Z. (2009). Reduction-sensitive reversibly crosslinked biodegradable

micelles for triggered release of doxorubicin. Macromolecular bioscience, 9(12), 1254-1261.

Heffernan, M. J., & Murthy, N. (2009). Disulfide-crosslinked polyion micelles for delivery of protein therapeutics. Annals of biomedical engineering, 37(10), 1993-2002.

Huang, J., Wu, F., Yu, Y., Huang, H., Zhang, S., & You, J. (2017). Lipoic acid based core cross-linked micelles for multivalent platforms: design, synthesis and application in bio-imaging and drug delivery. Organic & biomolecular chemistry, 15(22), 4798-4802.

A.Y.L. Tang, C.H. Lee, Y.M. Wang, and C.W. Kan. Effect of hydrophilic-lipophilic balance (HLB) values of PEG-based non-ionic surfactant on reverse micellar dyeing of cotton fibre with reactive dyes in non-aqueous medium. Fibers and Polymers, 19(4):894-904, 2018.

M. Pileni. Reverse micelles as microreactors. Journal of Physical Chemistry, 97(27):6961-6973, 1993.

M. Kotlarchyk, J.S. Huang, and S.H. Chen. Structure of AOT reversed micelles determined by small-angleneutron scattering. Journal of Physical Chemistry, 89(20):4382-4386, 1985.

S. Safran and L. Turkevich. Phase diagrams for microemulsions. Physical Reviews Letters, 50(24):1930-1933, 1983.

S.C. Owen, D.P.Y. Chan, and M.S. Shoichet. Polymeric micelle stability. Nano Today, 7:53-75, 2012.

G Tosi, L Costantino, F Rivasi, B Ruozi, E Leo, A V Vergoni, R Tacchi, A Bertolini, M A Vandelli, F Forni. Targeting the central nervous system: in vivo experiments with peptide-derivatized nanoparticles loaded with Loperamide and Rhodamine-123. J Control Release. 2007 Sep 11;122(1):1-9

## Claims

1. A polymeric reverse micelle for the delivery of an active pharmaceutical ingredient, wherein the interior of the reverse micelle is hydrophilic and is suitable to contain a water-soluble active pharmaceutical ingredient and the exterior of the reverse micelle is hydrophobic, wherein said reverse micelle comprises at least two polymers, each one having the following formula (I):

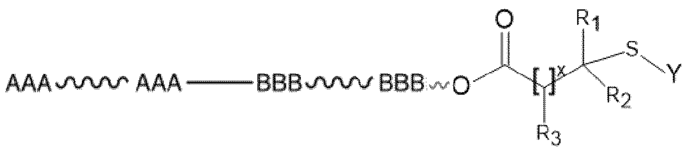

(I)

wherein

AAA ~~ AAA is an hydrophilic polymer covalently linked to BBB ~~ BBB which is an hydrophobic polymer,
R1, R2, R3, the same or different from each other, are selected from hydrogen, $C_{1-20}$ alkyl and $C_{1-20}$alkyl-O-$C_{1-20}$alkyl, preferably they are independently selected from methyl, ethyl, n-propyl or isopropyl;
x is 1 or 2;
Y is an alkyl chain substituted with at least two molecules of general formula (A):

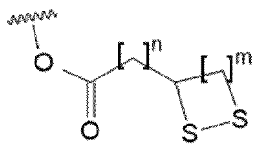

(A)

wherein n is comprised between 0 and 6 and
m is comprised between 0 and 5;
said Y alkyl chain being optionally terminally substituted with SH;
and wherein said at least two polymers of formula (I) are linked through disulfide bonds between the thiol groups

formed upon reduction of the -S-S- linkage in molecule (A),
wherein the percentage of thiol groups involved in inter-polymer disulfide bonds with respect to the total of the thiol groups present in molecule (A) is comprised between 70 and 100%, preferably between 90 and 100%.

**2.** The micelle according to claim 1 wherein m is 2.

**3.** The micelle according to anyone of claims 1-2 wherein Y is substituted with between two and four molecules of formula (A).

**4.** The micelle according to anyone of claims 1-3 wherein the hydrophilic polymer is polyethylene glycol and/or the hydrophobic polymer is poly (lactide-co-glycolide).

**5.** The micelle according to anyone of claims 1-4 wherein all the thiol groups of the molecules of formula (A) are involved in inter-polymer disulfide bonds.

**6.** The micelle according to anyone of claims 1-5 further comprising at least one water-soluble pharmaceutical active ingredient.

**7.** The micelle according to claim 6 wherein said pharmaceutical ingredient is a macromolecule.

**8.** The micelle according to claim 6 or 7 wherein said pharmaceutical ingredient is for the treatment of at least one disorder or condition selected from the following group: leukodystrophies, diseases of the central nervous system, such as neurodegenerative diseases, and lysosomal storage disorders, preferably it is a pharmaceutical ingredient for the treatment of a disorder of the central nervous system selected from brain tumor, Alzheimer disease and Parkinson disease.

**9.** The micelle according to anyone of claims 6-8 wherein said pharmaceutical ingredient is selected from:

- a protein, preferably a recombinant protein or a cytokine, such as DNL310, Filgrastim, JR-141, JR-171, Migalastat or NKTR-214;
- a peptide, such as GV1001, rh-Endostatine or Sargramostim;
- an enzyme, preferably selected from the group consisting of: Avalglucosidase, Cipaglucosidase Alfa, Elosulfase, Galsulfase, Iduronidase, Idursulfase, Imiglucerase, Laronidase, N-acetylgalactosamine 6-sulfatase, Taliglucerase Alfa, Velaglucerase alfa, Vestronidase alfa, α-galactosidase A (a-GAL), α-glucosidase (GAA), α-N-acetylglucosaminidase, β-Glucocerebrosidase (GCase), and galactosylceramidase (GALC);
- a nucleic acid, such as those used in gene therapy, for example ABO-101, ABO-102, AT845, AVR-RD-02, AXO-AAV-GM2, FBX-101, OTL-200, PBKR03, PR001, RGX-111, RGX-121, SPK-3006 or TSHA-101;
- an antibody, preferably selected from the group consisting of: Adalimumab, Bepranemab, Bevacizumab, Camrelizumab, Cetuximab, Durvalumab, Ipilimumab, m266, Magrolimab, Natalizumab, Nivolumab, Omburtamab, Panitumumab, Pembrolizumab, Pepinemab, Pritumumab, Rituximab, Sotigalimab, TB006 and Trastuzumab.

**10.** The micelle according to anyone of claims 1-9 wherein the micelle further comprises one or more polymers of formula (III)

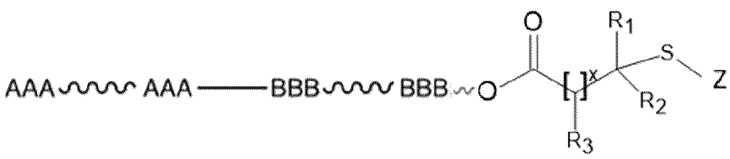

(III)

wherein AAA ∿ AAA, BBB ∿ BBB, R1, R2, R3 and x are as defined in claim 1, and wherein Z is an alkyl chain substituted with at least two hydroxyl groups and terminally conjugated to at least one targeting molecule, optionally further comprising a linker L between the alkyl chain Z and the conjugated targeting molecule, said linker L being

preferably a molecule bearing at least one alkylidene and/or vinyl group.

**11.** The micelle of claim 10 wherein said targeting molecule is selected from a peptide, a nucleic acid, such as a DNA or a RNA molecule, preferably an oligonucleotide or a polynucleotide, a protein, and any synthetic functional unit.

**12.** The micelle of claim 11 wherein said targeting molecule is a peptide selected from Angiopep-2 (having sequence TFFYGGSRGKRNNFKTEEYG; SEQ ID N.1), g7 peptide (GF(D-)TGFLS(O-b-D-glucose); SEQ ID N.2) and Tf2 peptide (having sequence GGGHKYLRW; SEQ ID N.3).

**13.** A process for obtaining a polymeric reverse micelle, wherein the interior of the reverse micelle is hydrophilic and is suitable to contain a water-soluble active pharmaceutical ingredient and the exterior of the reverse micelle is hydrophobic, comprising at least the following steps:

a) reacting at least two polymers, each polymer comprising a hydrophilic polymer covalently linked to an hydrophobic polymer, said hydrophobic polymer provided with a terminal hydroxyl group, with an alkenyl halide thus obtaining polymers with an - ene terminal functional group;
b) reacting the obtained polymers with a thiopolyol to obtain polymers with at least two hydroxyl lateral groups;
c) conjugating each of said hydroxyl lateral groups of the polymers with a cyclic disulfide of formula (A')

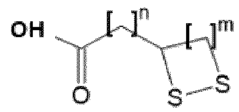

(A')

wherein n is comprised between 0 and 6 and m is comprised between 0 and 5, obtaining polymers with terminal rings having reducible disulfide bridges;
d) optionally adding one or more polymers obtained in step a) conjugated with a targeting molecule;
e) partially reducing said polymers obtained in step c) by adding a reducing agent to obtain polymers with free reduced thiol groups, which activate a thiol-disulphide exchange with non-reduced polymers having reducible terminal rings thus forming linear disulphide bonds between the polymers and obtaining a reverse micelle, wherein at the end of step e) the percentage of thiol groups involved in inter-polymer disulfide bonds with respect to the total of the thiol groups present in the cyclic disulfide groups is comprised between 70 and 100%, preferably between 90 and 100%.

**14.** A pharmaceutical composition comprising the reverse micelle of anyone of claims 1-12 loaded with at least one water-soluble pharmaceutical ingredient, and further comprising at least one pharmaceutically acceptable vehicle and/or excipient.

**15.** A polymer of formula (I)

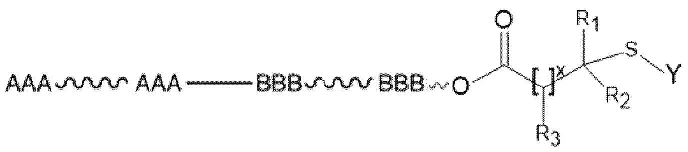

(I)

wherein

AAA ∿∿ AAA is an hydrophilic polymer covalently linked to BBB ∿∿ BBB which is an hydrophobic polymer,
R1, R2, R3, the same or different from each other, are selected from hydrogen, $C_{1-20}$ alkyl and $C_{1-20}$alkyl-O-$C_{1-20}$alkyl, preferably they are independently selected from methyl, ethyl, n-propyl or isopropyl;
x is 1 or 2;
Y is an alkyl chain substituted with at least two molecules of general formula (A):

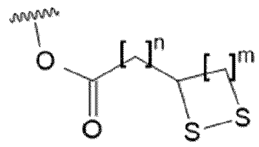

(A)

wherein n is comprised between 0 and 6 and m is comprised between 0 and 5; said Y alkyl chain being optionally terminally substituted with SH.

## Patentansprüche

**1.** Polymere reverse Mizelle für die Abgabe eines pharmazeutischen Wirkstoffs, wobei das Innere der reversen Mizelle hydrophil und geeignet ist, einen wasserlöslichen pharmazeutischen Wirkstoff zu enthalten, und das Äußere der reversen Mizelle hydrophob ist, wobei die reverse Mizelle mindestens zwei Polymere umfasst, wobei jedes die folgende Formel (I) aufweist

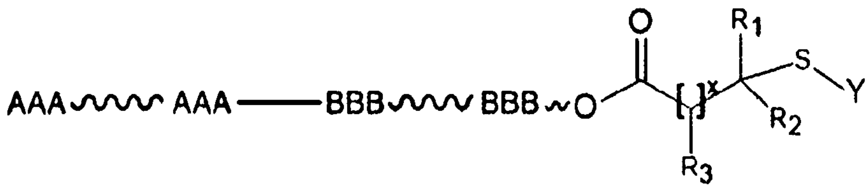

(I)

wobei

AAA ∿∿ AAA ein hydrophiles Polymer ist, das kovalent mit BBB ∿∿ BBB verknüpft ist, das ein hydrophobes Polymer ist,
R1, R2, R3 gleich oder verschieden voneinander sind, unter Wasserstoff, $C_{1-20}$-Alkyl und $C_{1-20}$-Alkyl-O-$C_{1-20}$-Alkyl ausgewählt sind, wobei sie bevorzugt unabhängig unter Methyl, Ethyl, n-Propyl oder Isopropyl ausgewählt sind;
x 1 oder 2 beträgt;
Y eine Alkylkette ist, die mit mindestens zwei Molekülen der allgemeinen Formel (A) substituiert ist:

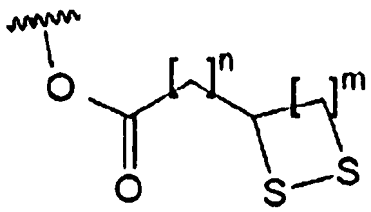

(A)

wobei n zwischen 0 und 6 liegt und
m zwischen 0 und 5 liegt;
wobei die Y-Alkylkette wahlweise endständig mit SH substituiert ist;
und wobei die mindestens zwei Polymere der Formel (I) durch Disulfidbindungen zwischen den Thiolgruppen verknüpft sind, die auf die Reduktion der -S-S-Verknüpfung in Molekül (A) hin gebildet sind,
wobei der Prozentsatz von Thiolgruppen, die in Zwischenpolymer-Disulfidbindungen involviert sind, mit Bezug auf die Gesamtheit der Thiolgruppen, die in dem Molekül (A) vorliegen, zwischen 70 und 100 %, bevorzugt zwischen 90 und 100 %, liegt.

**2.** Mizelle nach Anspruch 1, wobei m 2 beträgt.

**3.** Mizelle nach einem der Ansprüche 1-2, wobei Y mit zwischen zwei und vier Molekülen der Formel (A) substituiert ist.

**4.** Mizelle nach einem der Ansprüche 1-3, wobei das hydrophile Polymer Polyethylenglykol ist und/oder das hydrophobe Polymer Poly(lactid-co-glycolid) ist.

**5.** Mizelle nach einem der Ansprüche 1-4, wobei alle der Thiolgruppen der Moleküle der Formel (A) in Zwischenpolymer-Disulfidbindungen involviert sind.

**6.** Mizelle nach einem der Ansprüche 1-5, ferner mindestens einen wasserlöslichen pharmazeutischen Wirkstoff umfassend.

**7.** Mizelle nach Anspruch 6, wobei der pharmazeutische Bestandteil ein Makromolekül ist.

**8.** Mizelle nach Anspruch 6 oder 7, wobei der pharmazeutische Bestandteil zur Behandlung von mindestens einem Leiden oder Zustand dient, das/der aus der folgenden Gruppe ausgewählt ist: Leukodystrophien, Erkrankungen des Zentralnervensystems wie beispielsweise neurodegenerativen Erkrankungen und lysosomalen Speicherkrankheiten, wobei er bevorzugt ein pharmazeutischer Bestandteil für die Behandlung eines Leidens des Zentralnervensystems ist, das unter Hirntumor, Alzheimer-Krankheit und Parkinsonkrankheit ausgewählt wird.

**9.** Mizelle nach einem der Ansprüche 6-8, wobei der pharmazeutische Bestandteil ausgewählt wird unter:

- einem Protein, bevorzugt einem rekombinanten Protein oder einem Zytokin, wie beispielsweise DNL310, Filgrastim, JR-141, JR-171, Migalastat oder NKTR-214;
- einem Peptid, wie beispielsweise GV1001, rh-Endostatin oder Sargramostim;
- einem Enzym, das bevorzugt aus der Gruppe ausgewählt wird bestehend aus: Avalglucosidase, Cipaglucosidase Alfa, Elosulfase, Galsulfase, Iduronidase, Idursulfase, Imiglucerase, Laronidase, N-Acetylgalactosamin 6-sulfatase, Taliglucerase Alfa, Velaglucerase alfa, Vestronidase alfa, $\alpha$-Galactosidase A (a-GAL), $\alpha$-Glucosidase (GAA), $\alpha$-N-Acetylglucosaminidase, $\beta$-Glucocerebrosidase (GCase) und Galactosylceramidase (GALC);
- einer Nucleinsäure, wie beispielsweise denjenigen, die bei der Gentherapie verwendet werden, beispielsweise ABO-101, ABO-102, AT845, AVR-RD-02, AXO-AAV-GM2, FBX-101, OTL-200, PBKR03, PR001, RGX-111, RGX-121, SPK-3006 oder TSHA-101;
- einem Antikörper, der bevorzugt aus der Gruppe ausgewählt wird bestehend aus: Adalimumab, Bepranemab, Bevacizumab, Camrelizumab, Cetuximab, Durvalumab, Ipilimumab, m266, Magrolimab, Natalizumab, Nivolumab, Omburtamab, Panitumumab, Pembrolizumab, Pepinemab, Pritumumab, Rituximab, Sotigalimab, TB006 und Trastuzumab.

**10.** Mizelle nach einem der Ansprüche 1-9, wobei die Mizelle ferner ein oder mehrere Polymere der Formel (III) umfasst

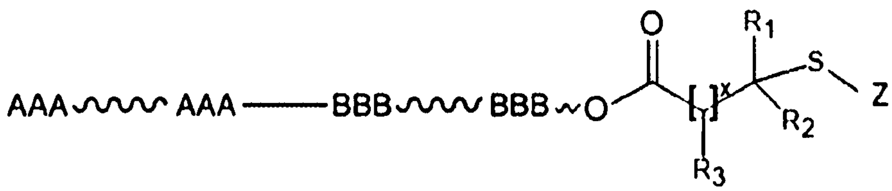

(III)

wobei AAA∿∿AAA , BBB∿∿BBB, R1, R2, R3 und x die Definition in Anspruch 1 aufweisen und wobei Z eine Alkylkette ist, die mit mindestens zwei Hydroxylgruppen substituiert und endständig an mindestens ein Targetingmolekül konjugiert ist, das wahlweise ferner einen Verknüpfer L zwischen der Alkylkette Z und dem konjugierten Targetingmolekül umfasst, wobei der Verknüpfer L bevorzugt ein Molekül ist, das mindestens eine Alkyliden- und/oder eine Vinylgruppe trägt.

**11.** Mizelle nach Anspruch 10, wobei das Targetingmolekül unter einem Peptid, einer Nucleinsäure wie beispielsweise einem DNA- oder einem RNA-Molekül, bevorzugt einem OligoNucleotid oder einem PolyNucleotid, einem Protein und irgendeiner synthetischen funktionellen Einheit ausgewählt wird.

**12.** Mizelle nach Anspruch 11, wobei das Targetingmolekül ein Peptid ist, das ausgewählt wird unter Angiopep-2 (das die

Sequenz TFFYGGSRGKRNNFKTEEYG; SEQ ID NO: 1 aufweist), g7-Peptid (GF(D-)TGFLS(O-b-D-Glucose); SEQ ID NO: 2) und Tf2-Peptid (das die Sequenz GGGHKYLRW; SEQ ID NO: 3 aufweist).

**13.** Verfahren zum Erhalten einer polymeren reversen Mizelle, wobei das Innere der reversen Mizelle hydrophil und geeignet ist, einen wasserlöslichen pharmazeutischen Wirkstoff zu enthalten, und das Äußere der reversen Mizelle hydrophob ist, umfassend mindestens die folgenden Schritte:

a) Reagieren von mindestens zwei Polymeren, wobei jedes Polymer ein hydrophiles Polymer umfasst, das kovalent mit einem hydrophoben Polymer verknüpft ist, wobei das hydrophobe Polymer mit einer endständigen Hydroxylgruppe ausgestattet ist, wobei ein Alkenylhalogenid so Polymere mit einer endständigen funktionellen -En-Gruppe erhält;
b) Reagieren der erhaltenen Polymere mit einem Thiopolyol, um Polymere mit mindestens zwei Hydroxylseitengruppen zu erhalten;
c) Konjugieren jeder der Hydroxylseitengruppen der Polymere mit einem cyclischen Disulfid der Formel (A')

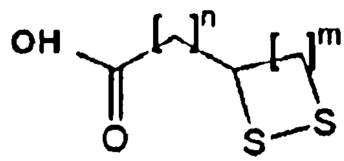

(A)

wobei n zwischen 0 und 6 liegt und m zwischen 0 und 5 liegt,
Erhalten von Polymeren mit endständigen Ringen, die reduzierbare Disulfidbrücken aufweisen;

d) wahlweise Zusetzen eines oder mehrere Polymere, die in Schritt a) erhalten worden sind, mit einem Targetingmolekül konjugiert;
e) teilweise Reduzieren der Polymere, die in Schritt c) erhalten worden sind, durch Zusetzen eines Reduktionsmittels, um Polymere mit freien reduzierten Thiolgruppen zu erhalten, die einen Thioldisulfidaustausch mit nichtreduzierten Polymeren aktivieren, die reduzierbare endständigen Ringe aufweisen, wodurch lineare Disulfidbindungen zwischen den Polymeren gebildet werden und eine reverse Mizelle erhalten wird,

wobei am Ende von Schritt e) der Prozentsatz von Thiolgruppen, die in Zwischenpolymer-Disulfidbindungen involviert sind, mit Bezug auf die Gesamtheit der Thiolgruppen, die in den cyclischen Disulfidgruppen vorliegen, zwischen 70 und 100 %, bevorzugt zwischen 90 und 100 %, liegt.

**14.** Pharmazeutische Zusammensetzung umfassend die reverse Mizelle nach einem der Ansprüche 1-12, die mit mindestens einem wasserlöslichen pharmazeutischen Bestandteil beladen ist und ferner mindestens ein pharmazeutisch akzeptables Vehikel und/oder einen pharmazeutisch akzeptablen Trägerstoff umfasst.

**15.** Polymer der Formel (I)

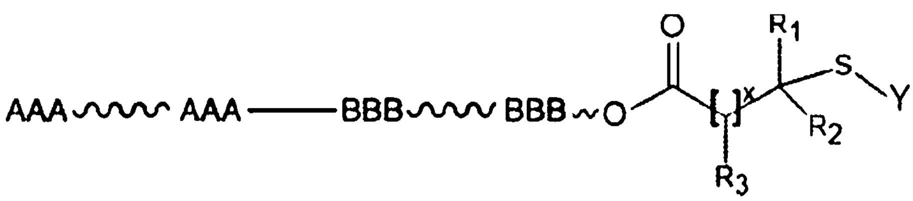

(I)

wobei

AAA ∿∿AAA ein hydrophiles Polymer ist, das kovalent mit BBB ∿∿BBB verknüpft ist, das ein hydrophobes Polymer ist,
R1, R2, R3 gleich oder verschieden voneinander und unter Wasserstoff, $C_{1-20}$-Alkyl und $C_{1-20}$-Alkyl-O-$C_{1-20}$-Al-

kyl ausgewählt sind, wobei sie bevorzugt unabhängig unter Methyl, Ethyl, n-Propyl oder Isopropyl ausgewählt sind;
x 1 oder 2 beträgt;
Y eine Alkylkette ist, die mit mindestens zwei Molekülen der allgemeinen Formel (A) substituiert ist:

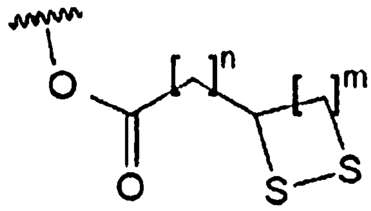

(A)

wobei n zwischen 0 und 6 liegt und m zwischen 0 und 5 liegt;
wobei die Y-Alkylkette wahlweise endständig mit SH substituiert ist.

## Revendications

**1.** Micelle inverse polymère pour la délivrance d'un ingrédient pharmaceutique actif, dans lequel l'intérieur du micelle inverse est hydrophile et est approprié pour contenir un ingrédient pharmaceutique actif hydrosoluble et l'extérieur du micelle inverse est hydrophobe, ledit micelle inverse comprenant au moins deux polymères, chacun ayant la formule (I) suivante

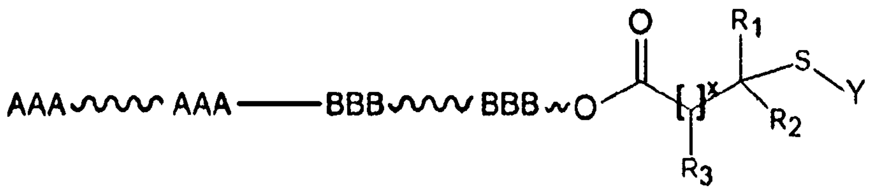

(I)

dans laquelle

AAA∿AAA est un polymère hydrophile lié de manière covalente à BBB∿BBB qui est un polymère hydrophobe,
R1, R2, R3, identiques ou différents les uns aux autres, sont choisis parmi un hydrogène, un alkyle en $C_1$ à $C_{20}$ et un alkyle en $C_1$ à $C_{20}$-O-alkyle en $C_1$ à $C_{20}$, de préférence ils sont choisis indépendamment parmi un méthyle, un éthyle, un n-propyle ou un isopropyle
x vaut 1 ou 2 ;
Y est une chaîne alkyle substituée avec au moins deux molécules de formule générale (A) :

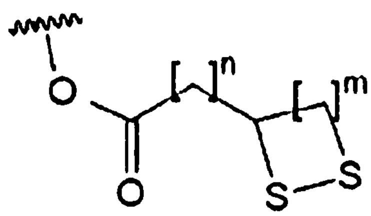

(A)

dans laquelle n est compris entre 0 et 6 et m est compris entre 0 et 5 ;
ladite chaîne alkyle Y étant éventuellement substituée de manière terminale avec SH ;
et lesdits au moins deux polymères de formule (I) étant liés par des liaisons disulfure entre les groupes thiol formés après réduction de la liaison -S-S- dans la molécule (A),

le pourcentage de groupes thiol impliqués dans des liaisons disulfure inter-polymère par rapport au total des groupes thiol présents dans la molécule (A) est compris entre 70 et 100 %, de préférence entre 90 et 100 %.

**2.** Micelle selon la revendication 1 dans lequel m vaut 2.

**3.** Micelle selon l'une quelconque des revendications 1 à 2 dans lequel Y est substitué avec entre deux et quatre molécules de formule (A).

**4.** Micelle selon l'une quelconque des revendications 1 à 3 dans lequel le polymère hydrophile est le polyéthylène glycol et/ou le polymère hydrophobe est le poly(lactide-co-glycolide) .

**5.** Micelle selon l'une quelconque des revendications 1 à 4 dans lequel tous les groupes thiol des molécules de formule (A) sont impliqués dans des liaisons disulfure inter-polymère.

**6.** Micelle selon l'une quelconque des revendications 1 à 5 comprenant en outre au moins un ingrédient actif pharmaceutique hydrosoluble.

**7.** Micelle selon la revendication 6 dans lequel ledit ingrédient pharmaceutique est une macromolécule.

**8.** Micelle selon la revendication 6 ou 7 dans lequel ledit ingrédient pharmaceutique est pour le traitement d'au moins un trouble ou état choisi dans le groupe suivant : leucodystrophies, maladies du système nerveux central, telles que les maladies neurodégénératives et les troubles du stockage lysosomal, de préférence c'est un ingrédient pharmaceutique pour le traitement d'un trouble du système nerveux central choisi parmi une tumeur du cerveau, la maladie d'Alzheimer et la maladie de Parkinson.

**9.** Micelle selon l'une quelconque des revendications 6 à 8 dans lequel ledit ingrédient pharmaceutique est choisi parmi :

- une protéine, de préférence une protéine recombinante telle que DNL310, le filgrastim, JR-141, JR-171, le migalastat ou NKTR-214,
- un peptide tel que GV1001, la rh-endostatine ou le sargramostim,
- une enzyme, de préférence choisie dans le groupe constitué par l'avaglucosidase, la cipaglucosidase alfa, l'élosulfase, la galsulfase, l'iduronidase, l'iidursulfase, l'imiglucérase, la laronidase, la N-acétylgalactosamine 6-sulfatase, la taliglucérase alfa, la vélaglucérase alfa, la vestronidase alfa, l'$\alpha$-galactosidase A (a-GAL), l'$\alpha$-glucosidase (GAA), l'$\alpha$-N-acétylglucosaminidase, la $\beta$-glucocérébrosidase (GCase) et la galactosylcéramidase (GALC) ;
- un acide nucléique tel que ceux utilisés en thérapie génique, par exemple ABO-101, ABO-102, AT845, AVR-RD-02, AXO-AAV-GM2, FBX-101, OTL-200, PBKR03, PR001, RGX-111, RGX-121, SPK-3006 ou TSHA-101 ;
- un anticorps choisi de préférence dans le groupe constitué par : l'adalimumab, lle bépranémab, le bévacizumab, la camrélizumab, le cétuximab, le durvalumab, l'ipilimumab, m266, le magrolimab, le natalizumab, le nivolumab, l'omburtamab, le panitumumab, le pembrolizumab, le pépinémab, le pritumumab, le rituximab, le sotigalimab, TB006 et le trastuzumab.

**10.** Micelle selon l'une quelconque des revendications 1 à 9 dans lequel le micelle comprend en outre un ou plusieurs polymères de formule (III)

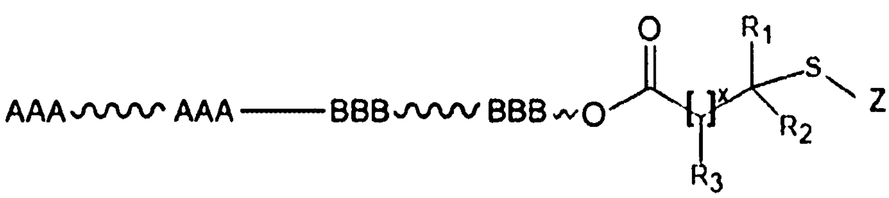

(III)

dans laquelle AAA∿∿∿AAA, BBB∿∿∿BBB, R1, R2, R3 et x sont tels que définis dans la revendication 1 et Z étant une chaîne alkyle substituée avec au moins deux groupes hydroxyle et conjuguée de manière terminale à au moins une molécule de ciblage, éventuellement comprenant en outre un lieur L entre le Z de la chaîne alkyle et la molécule de ciblage conjuguée, ledit lieur L étant de préférence une molécule portant au moins un groupe alkylidène et/ou vinyle.

**11.** Micelle selon la revendication 10 dans lequel ladite molécule de ciblage est choisie parmi un peptide, un acide nucléique, tel qu'une molécule d'ADN ou d'ARN, de préférence un oligonucléotide ou un polynucléotide, une protéine et une quelconque unité fonctionnelle synthétique.

**12.** Micelle selon la revendication 11 dans lequel ladite molécule de ciblage est un peptide choisi parmi l'angiopep-2 (ayant la séquence TFFYGGSRGKRNNFKTEEYG SEQ ID n° 1), le peptide g7 (GF(D-)TGFLS(O-b-D-glucose) : SEQ ID n° 2 et le peptide Tf2 (ayant la séquence GGGHKYLRW, SEQ ID n° 3).

**13.** Procédé d'obtention d'un micelle inverse polymère, dans lequel l'intérieur du micelle inverse est hydrophile et est approprié pour contenir un ingrédient pharmaceutique actif hydrosoluble et l'extérieur du micelle inverse est hydrophobe comprenant au moins les étapes suivantes

a) réaction d'au moins deux polymères, chaque polymère comprenant un polymère hydrophile lié de manière covalente à un polymère hydrophobe, ledit polymère hydrophobe étant fourni avec un groupe hydroxyle terminal, avec un halogénure d'alcényle obtenant ainsi des polymères avec un groupe fonctionnel terminal -ène,
b) la réaction des polymères obtenus avec un thiopolyol pour obtenir des polymères avec au moins deux groupes latéraux hydroxyle ;
c) la conjugaison de chacun desdits groupes latéraux hydroxyle des polymères avec un disulfure cyclique de formule (A')

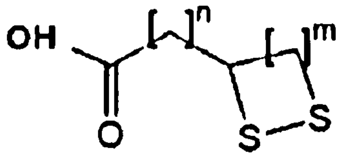

(A)

dans laquelle n est compris entre 0 et 6 et m est compris entre 0 et 5, obtenant des polymères avec des cycles terminaux ayant des ponts disulfure réductibles,
d) éventuellement l'addition d'un ou de plusieurs polymères obtenus dans l'étape a) conjugués avec une molécule de ciblage,
e) partiellement réduire lesdits polymères obtenus dans l'étape c) par addition d'un agent réducteur pour obtenir des polymères avec des groupes thiol réduits libres, qui activent un échange thiol-disulfure avec des polymères non réduits ayant des cycles terminaux réductibles formant ainsi des liaisons disulfure linéaires entre les polymères et obtenant un micelle inverse,

à la fin de l'étape e) le pourcentage de groupes thiol impliqués dans des liaisons disulfure inter-polymère par rapport au total des groupes thiol présents dans les groupes disulfure cycliques est compris entre 70 e 100 %, de préférence entre 90 et 100 %.

**14.** Composition pharmaceutique comprenant le micelle inverse selon l'une quelconque des revendications 1 à 12 chargé avec au moins un ingrédient pharmaceutique hydrosoluble et comprenant en outre au moins un véhicule et/ou excipient pharmaceutiquement acceptable.

**15.** Polymère de formule (I)

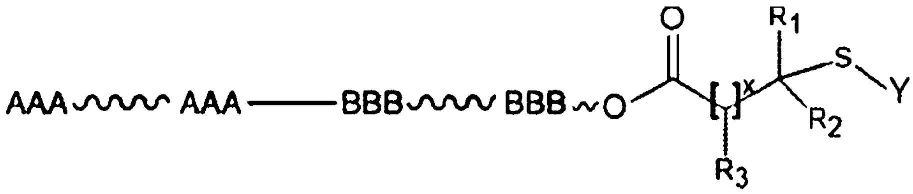

(I)

dans laquelle

AAA∿∿AAA est un polymère hydrophile lié de manière covalente à BBB∿∿BBB qui est un polymère hydrophobe,

R1, R2, R3, identiques ou différents les uns des autres, sont choisis parmi l'hydrogène, un alkyle en $C_1$ à $C_{20}$ et un alkyle en $C_1$ à $C_{20}$-O-alkyle en $C_1$ à $C_{20}$, de préférence ils sont choisis indépendamment parmi un méthyle, un éthyle, un n-propyle ou un iisopropyle,

x vaut 1 ou 2,

Y est une chaîne alkyle substituée avec au moins deux molécules de formule générale (A) :

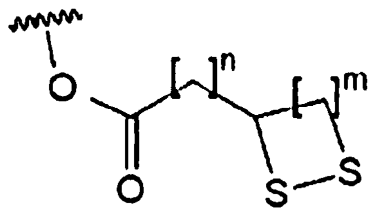

(A)

dans laquelle n est compris entre 0 et 6 et m est compris entre 0 et 5, ladite chaîne Y étant éventuellement substituée de manière terminale avec SH.

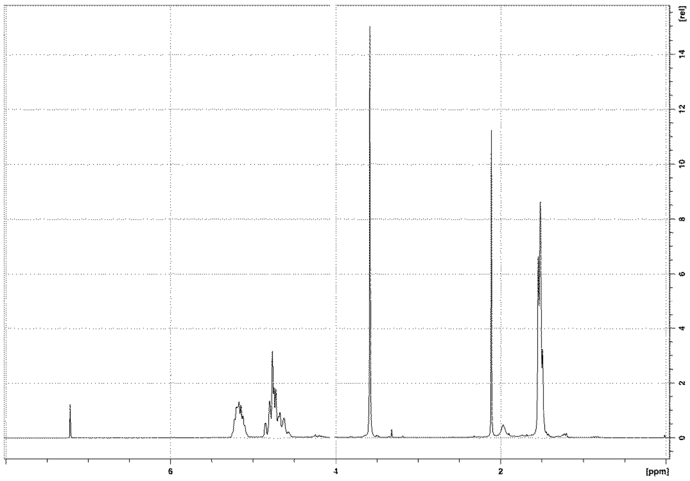


Figure 1

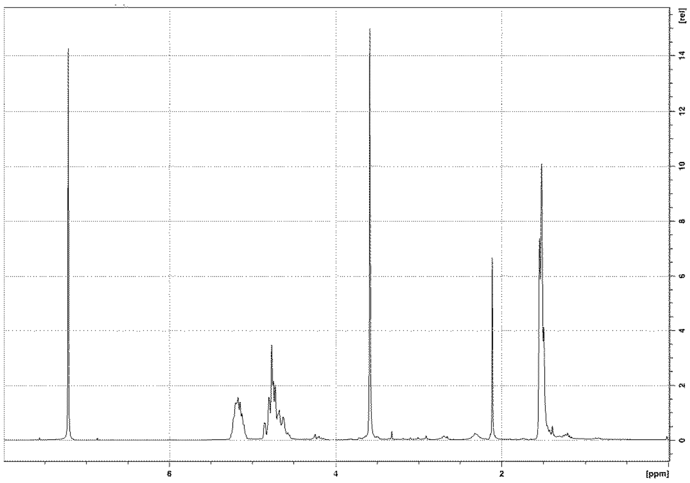


Figure 2

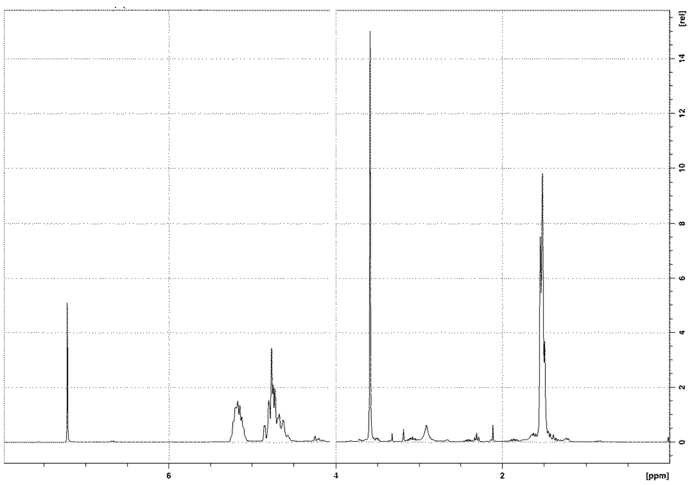


Figure 3

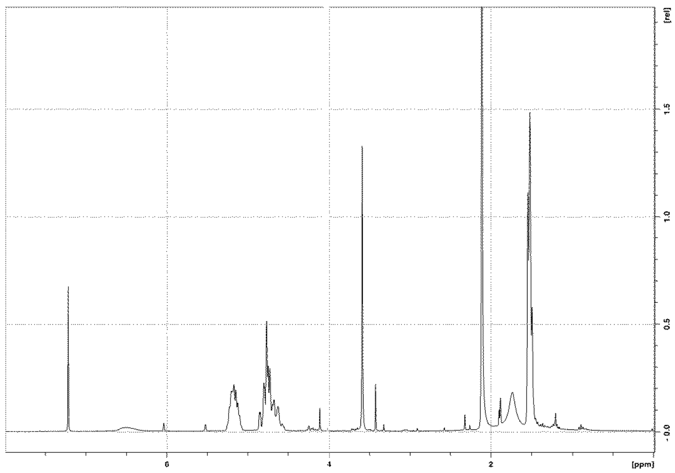


Figure 4

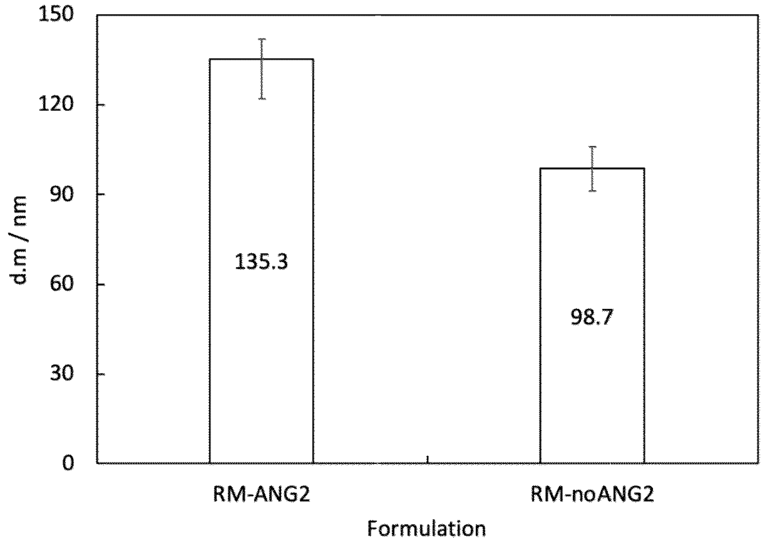


Figure 5

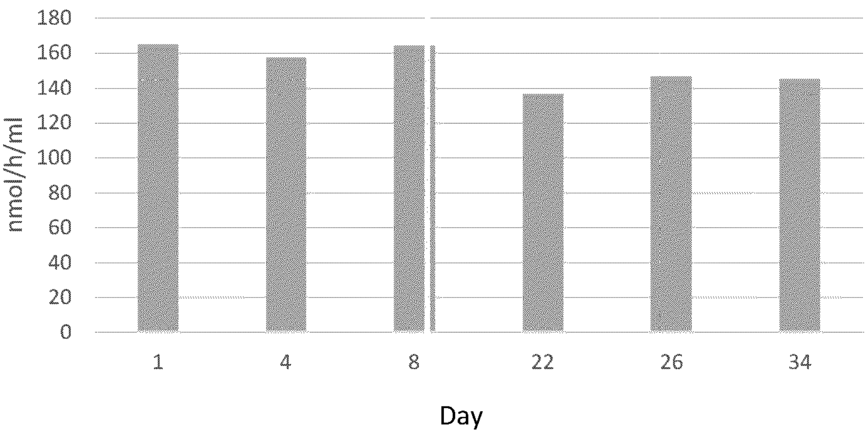


Figure 6

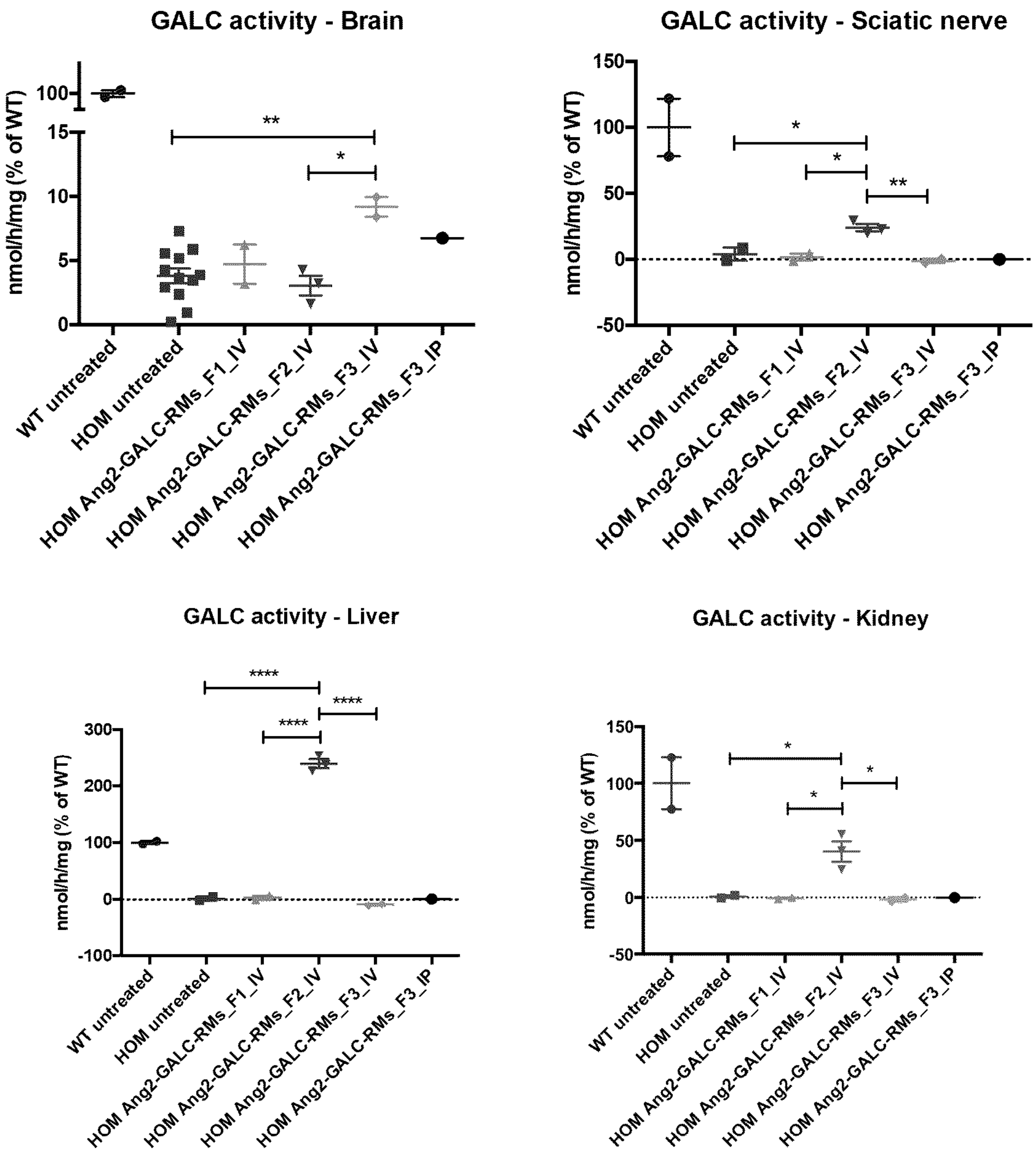
GALC activity - Brain
nmol/h/mg (% of WT)
100
15
10
5
0
**
*
WT untreated
HOM untreated
HOM Ang2-GALC-RMs_F1_IV
HOM Ang2-GALC-RMs_F2_IV
HOM Ang2-GALC-RMs_F3_IV
HOM Ang2-GALC-RMs_F3_IP
GALC activity - Sciatic nerve
nmol/h/mg (% of WT)
150
100
50
0
-50
*
*
**
WT untreated
HOM untreated
HOM Ang2-GALC-RMs_F1_IV
HOM Ang2-GALC-RMs_F2_IV
HOM Ang2-GALC-RMs_F3_IV
HOM Ang2-GALC-RMs_F3_IP
GALC activity - Liver
nmol/h/mg (% of WT)
300
200
100
0
-100
****
****
****
WT untreated
HOM untreated
HOM Ang2-GALC-RMs_F1_IV
HOM Ang2-GALC-RMs_F2_IV
HOM Ang2-GALC-RMs_F3_IV
HOM Ang2-GALC-RMs_F3_IP
GALC activity - Kidney
nmol/h/mg (% of WT)
150
100
50
0
-50
*
*
*
WT untreated
HOM untreated
HOM Ang2-GALC-RMs_F1_IV
HOM Ang2-GALC-RMs_F2_IV
HOM Ang2-GALC-RMs_F3_IV
HOM Ang2-GALC-RMs_F3_IP

Figure 7

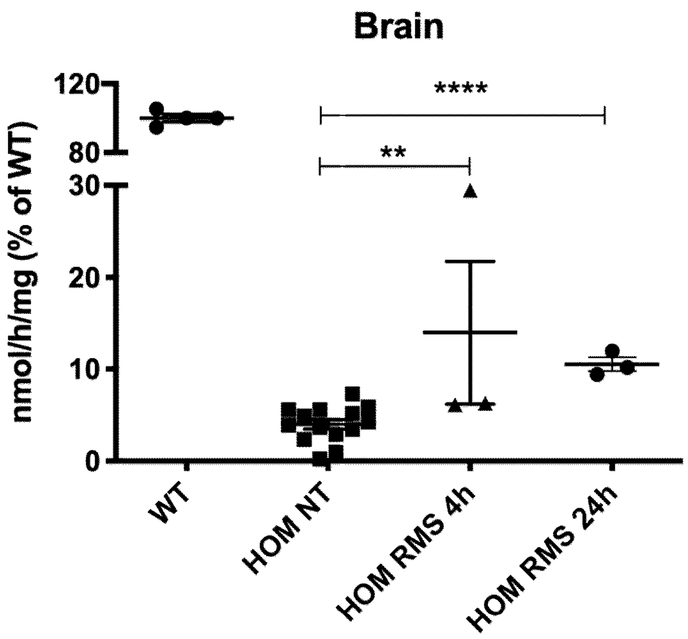


Figure 8

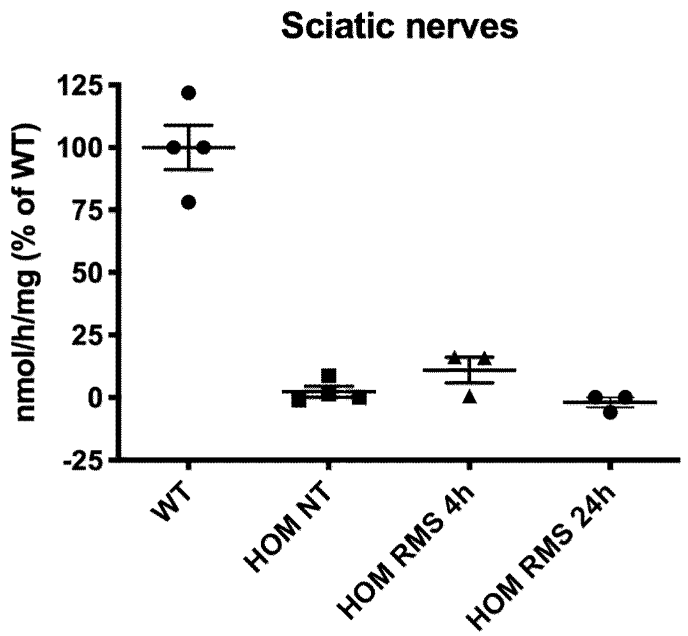


Figure 9

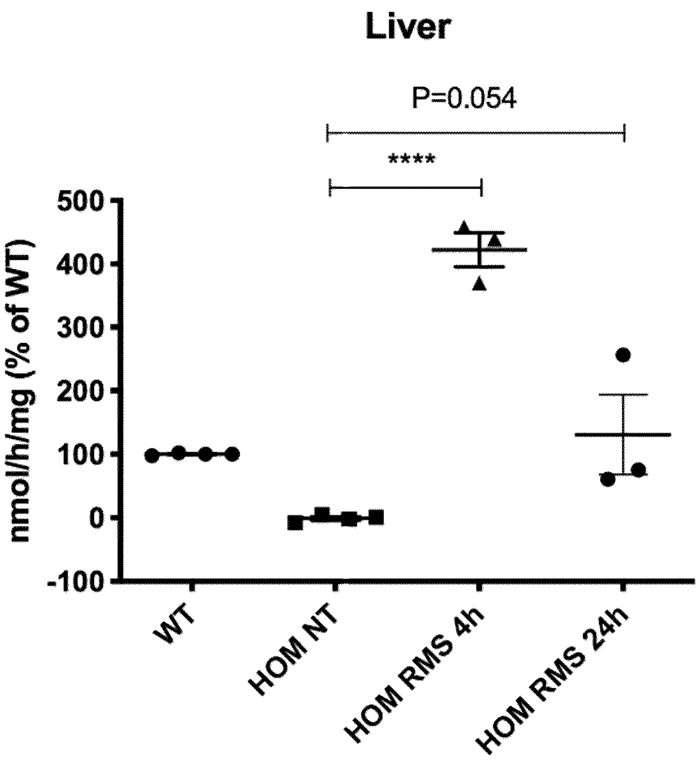


Figure 10

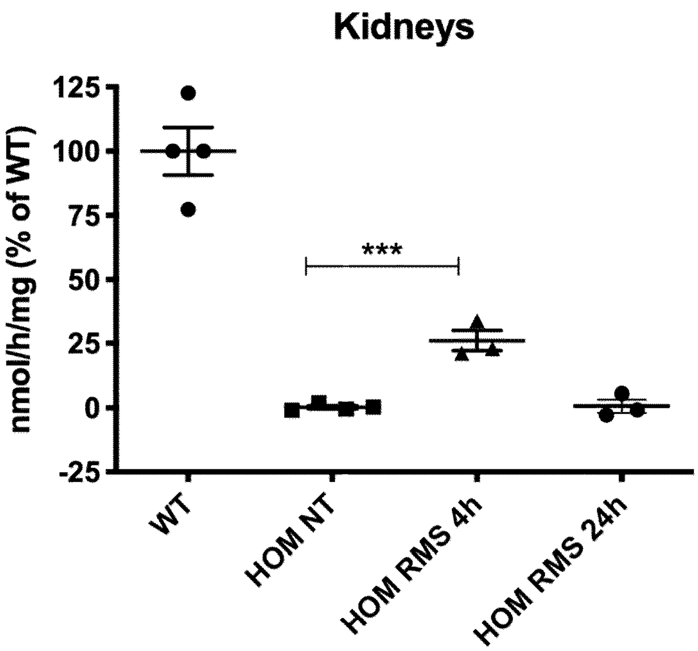


Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012105485 A1
- CN 104004199 A
- CN 103272237 A
- CN 110522720 A
- US 20100196482 A1
- WO 2021046078 A1


### Non-patent literature cited in the description


- Remington's Pharmaceutical Science. Mack Publishing Company, 1985
- **KOYAMATSU, Y.** ; **HIRANO, T.** ; **KAKIZAWA, Y.** ; **OKANO, F.** ; **TAKARADA, T.** ; **MAEDA, M.** pH-responsive release of proteins from biocompatible and biodegradable reverse polymer micelles. *Journal of Controlled Release*, 2014, vol. 173, 89-95
- **XU, Y.** ; **MENG, F.** ; **CHENG, R.** ; **ZHONG, Z.** Reduction-sensitive reversibly crosslinked biodegradable micelles for triggered release of doxorubicin. *Macromolecular bioscience*, 2009, vol. 9 (12), 1254-1261
- **HEFFERNAN, M. J.** ; **MURTHY, N.** Disulfide-crosslinked polyion micelles for delivery of protein therapeutics.. *Annals of biomedical engineering*, 2009, vol. 37 (10), 1993-2002
- **HUANG, J.** ; **WU, F.** ; **YU, Y.** ; **HUANG, H.** ; **ZHANG, S.** ; **YOU, J.** Lipoic acid based core cross-linked micelles for multivalent platforms: design, synthesis and application in bio-imaging and drug delivery.. *Organic & biomolecular chemistry*, 2017, vol. 15 (22), 4798-4802
- **A.Y.L. TANG** ; **C.H. LEE** ; **Y.M. WANG** ; **C.W. KAN.** Effect of hydrophilic-lipophilic balance (HLB) values of PEG-based non-ionic surfactant on reverse micellar dyeing of cotton fibre with reactive dyes in non-aqueous medium.. *Fibers and Polymers*, 2018, vol. 19 (4), 894-904
- **M. PILENI.** Reverse micelles as microreactors.. *Journal of Physical Chemistry*, 1993, vol. 97 (27), 6961-6973
- **M. KOTLARCHYK** ; **J.S. HUANG** ; **S.H. CHEN.** Structure of AOT reversed micelles determined by small-angleneutron scattering.. *Journal of Physical Chemistry*, 1985, vol. 89 (20), 4382-4386
- **S. SAFRAN** ; **L. TURKEVICH.** Phase diagrams for microemulsions.. *Physical Reviews Letters*, 1983, vol. 50 (24), 1930-1933
- **S.C. OWEN** ; **D.P.Y. CHAN** ; **M.S. SHOICHET.** Polymeric micelle stability.. *Nano Today*, 2012, vol. 7, 53-75
- **G TOSI** ; **L COSTANTINO** ; **F RIVASI** ; **B RUOZI** ; **E LEO, A V VERGONI** ; **R TACCHI** ; **A BERTOLINI** ; **M A VANDELLI** ; **F FORNI.** Targeting the central nervous system: in vivo experiments with peptide-derivatized nanoparticles loaded with Loperamide and Rhodamine-123.. *J Control Release.*, 11 September 2007, vol. 122 (1), 1-9